# EUROPEAN PATENT SPECIFICATION

(11) **EP 3 781 197 B1**
(45) Date of publication and mention of the grant of the patent: **10.09.2025**
(21) Application number: 19789302.7
(22) Date of filing: 18.04.2019
(51) Int. Cl.: A61K 38/50, A61K 47/60, C12N 9/82, A61P 35/02

(54) **VARIANT ASPARAGINASE POLYPEPTIDES FOR MEDICAL USE**
VARIANTE ASPARAGINASE-POLYPEPTIDE ZUR MEDIZINISCHEN VERWENDUNG
POLYPEPTIDES VARIANTS D'ASPARAGINASE À USAGE MÉDICAL

(30) Priority: 19.04.2018 US 201862660189 P
(43) Date of publication of application: 24.02.2021
(73) Proprietor: Elanco US Inc., Greenfield, IN 46140 (US)
(72) Inventor: ZHAN, Hangjun, Burlingame, California 94010 (US); NGUYEN, Lam, Burlingame, California 94010 (US); CHIN, Richard, Burlingame, California 94010 (US); LI, Shyr Jiann, Burlingame, California 94010 (US)
(74) Representative: Mewburn Ellis LLP
(86) International application number: PCT/US2019/028167
(87) International publication number: WO 2019/204636

(56) References cited:
- WO-A1-2008/110513
- WO-A1-2017/151707
- US-A1- 2015 010 522
- US-A1- 2016 060 613
- NGUYEN ET AL.: "Design and Characterization of Erwinia Chrysanthemi I-Asparaginase Variants with Diminished I-Glutaminase Activity", J BIOL CHEM, vol. 291, 27 June 2016 (2016-06-27), pages 17664 - 17676, XP055369173, DOI: 10.1074/jbc.M116.728485
- KOTZIA ET AL.: "Engineering thermal stability of L-asparaginase by in vitro directed evolution", FEBS J, vol. 276, 13 February 2009 (2009-02-13), pages 1750 - 1761, XP055369172, DOI: 10.1111/j.1742-4658.2009.06910.x

## Description

### RELATED APPLICATIONS

This application claims the benefit of priority of US Provisional Application No. 62/660,189, filed April 19, 2018.

### SEQUENCE LISTING

The present application is being filed along with a Sequence Listing in electronic format. The Sequence Listing is provided as a file entitled 2019-04-15_01157-0013-00PCT_Seq_List_ST25.txt created April 15, 2019, which is 42 Kb in size.

### FIELD

The present disclosure relates to variant asparaginase polypeptides having reduced immunogenicity and enhanced stability and pharmacokinetics, and methods of producing and using the same, for example, for treating acute lymphoblastic leukemia in humans and lymphoma in companion animals.

### BACKGROUND

In its native form, L-asparaginase (also referred to herein as asparaginase) is a bacterial enzyme composed of identical subunits (approximately 35 kDa each subunit) that catalyzes the conversion of L-asparagine to aspartic acid and ammonia. *Escherichia coli* or *E. coli* asparaginase may be a homo-dimer or a homo-tetramer and *Erwinia chrysanthemi* or *E*. *chrysanthemi* (also called *Dickeya dadantii*, *D. dadantii*, *Dickeya chrysanthemi*, *D. chrysanthemi*, etc.) asparaginase is a tetramer. Asparaginase is used to treat patients with acute lymphoblastic leukemia (ALL) and to treat dogs and cats with lymphoma. Some leukemic cells are not able to synthesize asparagine and depend on circulating asparagine for survival. As a therapeutic, asparaginase can deprive such cells of circulating asparagine leading to cell death. Normal cells are less affected by asparaginase because they are able to synthesize asparagine.

Unfortunately, hypersensitivity to asparaginase is a commonly reported adverse event and often requires termination of asparaginase treatment. Native *E. coli* asparaginase may be immunogenic when introduced to humans at therapeutic quantities. Antibodies to asparaginase can increase the elimination rate, and neutralization antibodies, if present, can limit the effectiveness of the enzyme.

### SUMMARY OF THE INVENTION

The invention is defined in the appended claims. This invention provides a variant asparaginase polypeptide comprising at least one amino acid substitution at an unpaired cysteine residue of a corresponding wildtype asparaginase polypeptide, wherein the at least one amino acid substitution comprises any amino acid other than cysteine.

In some embodiments, the variant asparaginase polypeptide comprises at least one cysteine substitution at an amino acid position. In other embodiments, the variant asparaginase polypeptide comprises at least one pair of cysteine substitutions at amino acid positions at or spatially near an immunogenic or antigenic site of the corresponding wildtype asparaginase polypeptide. In other embodiments, the variant asparaginase polypeptide comprises at least one partially embedded cysteine.In some embodiments, the variant asparaginase polypeptide lacks a leader sequence.

In some embodiments, the immunogenic or antigenic site in the wildtype asparaginase polypeptide elicits an immune response in a human or in a companion animal species. In some embodiments the companion animal species is a canine, feline, or equine.

In some embodiments, at least one cysteine is between 5% and 25%, between 5% and 20%, between 5% and 15%, from 5% and 10%, between 10% and 20%, between 20% and 30%, or between 10% and 30% surface-exposed, as determined by a standard protein modeling software.

In some embodiments, at least one cysteine is or the at least one pair of cysteines are 35 Angstroms (Å) or less (e.g. 30 Å or less, 25 Å or less, 20 Å or less, 15 Å or less, 10 Å or less, or 5 Å or less) from the immunogenic or antigenic site, as determined by three-dimensional protein structure analysis.

In some embodiments, the variant asparaginase polypeptide comprises an amino acid sequence that is at least 80%, at least 85%, at least 90%, at least 91%, at least 92%, at least 93%, at least 94%, at least 95%, at least 96%, at least 97%, at least 98%, or at least 99% identical to a wildtype asparaginase amino acid sequence, such as SEQ ID NO: 2, SEQ ID NO: 11, or SEQ ID NO: 14. In some embodiments, the wildtype asparaginase polypeptide is a wildtype *E. coli* asparaginase polypeptide. In some embodiments, the wildtype asparaginase polypeptide comprises SEQ ID NO: 2, SEQ ID NO: 11, or SEQ ID NO: 14.

In some embodiments, the immunogenic or antigenic site corresponds to amino acid position 55, 56, 57, 58, 114, 115, 116, 117, 118, 119, 201, 202, 203, 204, 205, 206, 207, 252, 253, 254, 255, 256, 257, or 258 of SEQ ID NO: 11. In some embodiments, at least one cysteine is located at a position corresponding to one or more amino acid position(s) selected from 51, 52, 118, 119, 196, 206, 285, and 311 of SEQ ID NO: 11. In some embodiments, each of the at least one pair of cysteines is selected from C1-C2 paired cysteine substitution(s) listed in Table 5, optionally at a position corresponding to amino acid positions 116 and 120, amino acid positions 196 and 200, or amino acid positions 225 and 252 of SEQ ID NO: 11. In some embodiments, the unpaired cysteine residue is located at a position corresponding to amino acid position 45, 67, and/or 242 of SEQ ID NO: 14.

In some embodiments, the variant asparaginase polypeptide comprising the amino acid sequence of SEQ ID NO: 12 or SEQ ID NO: 15.

In some embodiments, the variant asparaginase polypeptide is in monomeric, dimeric, or tetrameric form.

In some embodiments, the variant asparaginase polypeptide is modified, such as sialyated or pegylated, optionally thiol-pegylated or amine-pegylated.

Also provided are isolated nucleic acids encoding the variant asparaginase polypeptides of the invention.

Also provided are vectors encoding the isolated nucleic acids of the invention.

Also provided are host cells comprising the vectors of the invention. In some embodiments, the host cell is a prokaryotic cell, such as an *E. chrysanthemi* cell, an *E. coli* cell, or a *pseudomonas* cell. In other embodiments, the cell is a eukaryotic cell, such as a yeast cell.

Also provided are methods of producing a variant asparaginase polypeptide comprising culturing the host cell. In some embodiment, the method of producing a variant asparaginase polypeptide further comprises isolating the variant.

Also provided are pharmaceutical compositions comprising a variant asparaginase polypeptide of the invention and a pharmaceutically acceptable carrier.

The variant asparaginase polypeptides or pharmaceutical compositions comprising the variant asparaginase polypeptides of the invention may be used in a method of treatment wherein the polypeptide or composition is administered to a subject. In some embodiments, the variant asparaginase polypeptides or pharmaceutical compositions comprising the variant asparaginase polypeptides of the invention may be used in a method of treating lymphoma or an actuate lymphoblastic leukemia (ALL).

In some embodiments, the subject in need of treatment is a human or a companion animal species. In some embodiments, the companion animal species is canine, feline, or equine.

### BRIEF DESCRIPTION OF THE DRAWINGS

FIG. 1A is a table listing the wash and elution buffers used in the cation exchange purification of variant *E. chrysanthemi* asparaginase (SEQ ID NO: 5), as described in Example 4.
FIG. 1B is a non-reducing SDS-PAGE stained with Coomassie showing variant *E. chrysanthemi* asparaginase (SEQ ID NO: 5) after E. coli intracellular expression and purification.
FIG. 2A shows size exclusion chromatography results for a variant *E. chrysanthemi* asparaginase (SEQ ID NO: 5) and protein standard markers.
FIG 2B lists elution times from size exclusion chromatography of a variant *E. chrysanthemi* asparaginase (SEQ ID NO: 5) and protein standard markers.

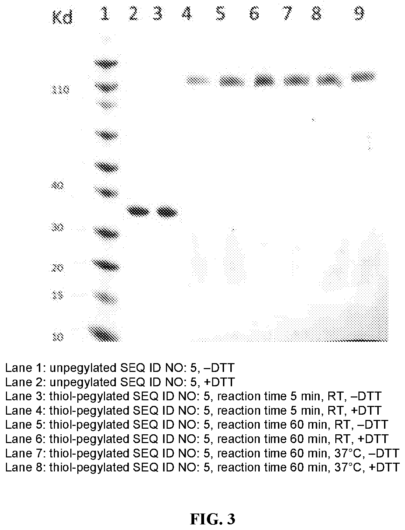

FIG. 3 shows an SDS-PAGE of a variant *E. chrysanthemi* asparaginase (SEQ ID NO: 5) unpegylated and thiol-pegylated at different reaction times (5 minutes and 60 minutes) and different reaction temperatures (room temperature and 37°C) under reducing (+DTT) and non-reducing (-DTT) conditions. Lane 1: unpegylated, -DTT; Lane 2: unpegylated, + DTT; Lane 3: thiol-pegylated, reaction time 5 minutes, RT, -DTT; Lane 4: thiol-pegylated, reaction time 5 minutes, RT, +DTT; Lane 5: thiol-pegylated, reaction time 60 minutes, RT, -DTT; Lane 6: thiol-pegylated, reaction time 60 minutes, RT, +DTT; Lane 7: thiol-pegylated, reaction time 60 minutes, 37 °C, -DTT; Lane 8: thiol-pegylated, reaction time 60 minutes, 37 °C, +DTT.
FIG. 4 illustrates a variant *E*. *coli* asparaginase polypeptide of SEQ ID NO: 12. The additional cysteine pair is identified.

### DESCRIPTION OF THE SEQUENCES

Table 1 provides a listing of certain sequences referenced herein.

| Table 1: Description of the Sequences | | |
|---|---|---|
| SEQ ID NO: | SEQUENCE | DESCRIPTION |
| 1 | MERWFKSLFVLVLFFVFTASAADKLPNIVI LATGGTIAGSAATGTQTTGYKAGALGVDTL INAVPEVKKLANVKGEQFSNMASENMTGDV VLKLSQRVNELLARDDVDGVVITHGTDTVE ESAYFLHLTVKSDKPVVFVAAMRPATAISA DGPMNLLEAVRVAGDKQSRGRGVMVVINDR IGSARYITKTNASTLDTFRANEEGYLGVII GNRIYYQNRIDKLHTTRSVFDVRGLTSLPK VDILYGYQDDPEYLYDAAIQHGVKGIVYAG MGAGSVSVRGIAGMRKALEKGVVVMRSTRT GNGIVPPDEELPGLVSDSLNPAHARILLML ALTRTSDPKVIQEYFHTY | *E. chrysanthemi* asparaginase precursor sequence |
| | | UniProtKB Accession No. P06608; CAA31239.1 L-asparagine aminohydrolase precursor (Dickeya chrysanthemi) |
| | | Strain NCPPB 1066 |
| 2 | ADKLPNIVILATGGTIAGSAATGTQTTGYK AGALGVDTLINAVPEVKKLANVKGEQFSNM ASENMTGDVVLKLSQRVNELLARDDVDGVV ITHGTDTVEESAYFLHLTVKSDKPVVFVAA MRPATAISADGPMNLLEAVRVAGDKQSRGR GVMVVINDRIGSARYITKTNASTLDTFRAN EEGYLGVIIGNRIYYQNRIDKLHTTRSVFD VRGLTSLPKVDILYGYQDDPEYLYDAAIQH GVKGIVYAGMGAGSVSVRGIAGMRKALEKG VVVMRSTRTGNGIVPPDEELPGLVSDSLNP AHARILLMLALTRTSDPKVIQEYFHTY | *E. chrysanthemi* asparaginase mature sequence |
| 3 | MADKLPNIVILATGGTIAGSAATGTQTTGY KAGALGVDTLINAVPEVKKLANVKGEQFSN MASENMTGDVVLKLSQRVNELLARDDVDGV VITHGTDTVEESAYFLHLTVKSDKPVVFVA AMRPATAISADGPMNLLEAVRVAGDKQSRG RGVMVVINDRIGSARYITKTNASTLDTFRA NEEGYLGVIIGNRIYYQNRIDKLHTTRSVF DVRGLTSLPKVDILYGYQDDPEYLYDAAIQ HGVKGIVYAGMGAGSVSVRGIAGMRKALEK GVVVMRSTRTGNGIVPPDEELPGLVSDSLN PAHARILLMLALTRTSDPKVIQEYFHTY | Variant *E. chrysanthemi* asparaginase with no leader sequence for intracellular expression |
| 4 | MADKLPNIVILATGGTIAGSAATGTQTTGY KAGALGVDTLICAVPEVKKLANVKGEQFSN MASENMTGDVVLCLSQRVNELLARDDVDGV VITHGTDTVEESAYFLHLTVKSDKPVVFVA AMRPATAISADGPMNLLEAVRVAGDKQSRG RGVMVVINDRIGSARYITKTNASTLDTFRA NEEGYLGVIIGNRIYYQNRIDKLHTTRSVF DVRGLTSLPKVDILYGYQDDPEYLYDAAIQ HGVKGIVYAGMGAGSVSVRGIAGMRCALEK GVVVMRSTRTGNGIVPPDEELPGLVSDSLN PAHARILLMLALTRTSDPKVIQEYFHTY | Variant *E. chrysanthemi* asparaginase mature sequence with 3 cysteine substitutions |
| | | N41C |
| | | K72C |
| | | K265C |
| 5 | MAHHHHHHHADKLPNIVILATGGTIAGSAA TGTQTTGYKAGALGVDTLICAVPEVKKLAN VKGEQFSNMASENMTGDVVLCLSQRVNELL ARDDVDGVVITHGTDTVEESAYFLHLTVKS DKPVVFVAAMRPATAISADGPMNLLEAVRV | Variant *E. chrysanthemi* asparaginase mature sequence with 3 cysteine substitutions and N-terminal polyHis tag |
| | AGDKQSRGRGVMVVINDRIGSARYITKTNA STLDTFRANEEGYLGVIIGNRIYYQNRIDK LHTTRSVFDVRGLTSLPKVDILYGYQDDPE YLYDAAIQHGVKGIVYAGMGAGSVSVRGIA GMRCALEKGVVVMRSTRTGNGIVPPDEELP GLVSDSLNPAHARILLMLALTRTSDPKVIQ EYFHTY | Intracellular expression of N-terminal polyHis_EW ASP |
| 6 | MAHHHHHHHADKLPNIVILATGGTIAGSAA TGTQTTGYKAGALGVDTLINAVPEVKKLAN VKGEQFSNMASENMTGDVVLKLSQRVNELL ARDDVDGVVITHGTDTVEESAYFLHLTVKS DKPVVFVAAMRPATAISADGPMNLLEAVRV AGDKQSRGRGVMVVINDRIGSARYITKTNA STLDTFRANEEGYLGVIIGNRIYYQNRIDK LHTTRSVFDVRGLTSLPKVDILYGYQDDPE YLYDAAIQHGVKGIVYAGMGAGSVSVRGIA GMRKALEKGVVVMRSTRTGNGIVPPDEELP GLVSDSLNPAHARILLMLALTRTSDPKVIQ EYFHTY | Variant *E. chrysanthemi* asparaginase sequence with N-terminal polyHis tag |
| | | Intracellular expression of N-terminal polyHis_EW ASP |
| 7 | MADKLPNIVILATGGTIAGSAATGTQTTGY KAGALGVDTLICAVPEVKKLANVKGEQFSN MASENMTGDVVLCLSQRVNELLARDDVDGV VITHGTDTVEESAYFLHLTVKSDKPVVFVA AMRPATAISADGPMNLLEAVRVAGDKQSRG RGVMVVINDRIGSARYITKTNASTLDTFRA NEEGYLGVIIGNRIYYQNRIDKLHTTRSVF DVRGLTSLPKVDILYGYQDDPEYLYDAAIQ HGVKGIVYAGMGAGSVSVRGIAGMRCALEK GVVVMRSTRTGNGIVPPDCELPGLVSDSLN PAHARILLMLALTRTSDPKVIQEYFHTY | Variant *E. chrysanthemi* asparaginase mature sequence with 4 cysteine substitutions |
| | | N41C |
| | | K72C |
| | | K265C |
| | | E288C |
| 8 | MADKLPNIVILATGGTIAGSAATGTQTTGY KAGALGVDTLIKAVPEVKKLANVKGEQFSN MASENMTGDVVLKLSQRVNELLARDDVDGV VITHGTDTVEESAYFLHLTVKSDKPVVFVA AMRPATAISADGPMNLLEAVRVAGDKQSRG RGVMVVINDRIGSARYITKTNASTLDTFRA NEEGYLGVIIGNRIYYQNRIDKLHTTRSVF DVRGLTSLPKVDILYGYQDDPEYLYDAAIQ HGVKGIVYAGMGAGSVSVRGIAGMRKALEK GVVVMRSTRTGNGIVPPDEELPGLVSDSLN PAHARILLMLALTRTSDPKVIQEYFHTY | Variant *E. chrysanthemi* asparaginase mature sequence with 1 lysine substitution |
| | | N41K |
| 9 | MADKLPNIVILATGGTIAGSAATGTQTTGY KAGALGVDTLIKAVPEVKKLANVKGEQFSN MASENMTGDVVLKLSQRVNELLARDDVDGV VITHGTDTVEESAYFLHLTVKSDKPVVFVA AMRPATAISADGPMNLLEAVRVAGDKQSRG RGVMVVINDRIGSARYITKTNASTLDTFRA NEEGYLGVIIGNRIYYQNRIDKLHTTRSVF DVRGLTSLPKVDILYGYQDDPEYLYDAAIQ HGVKGIVYAGMGAGSVSVRGIAGMRKALEK GVVVMRSTRTGNGIVPPDKELPGLVSDSLN PAHARILLMLALTRTSDPKVIQEYFHTY | Variant *E. chrysanthemi* asparaginase mature sequence with 2 lysine substitutions |
| | | N41K |
| | | E288K |
| | | |
| 10 | MEFFKKTALAALVMGFSGAALALPNITILA TGGTIAGGGDSATKSNYTVGKVGVENLVNA VPQLKDIANVKGEQVVNIGSQDMNDNVWLT LAKKINTDCDKTDGFVITHGTDTMEETAYF LDLTVKCDKPVVMVGAMRPSTSMSADGPFN LYNAVVTAADKASANRGVLVVMNDTVLDGR DVTKTNTTDVATFKSVNYGPLGYIHNGKID YQRTPARKHTSDTPFDVSKLNELPKVGIVY NYANASDLPAKALVDAGYDGIVSAGVGNGN LYKSVFDTLATAAKTGTAVVRSSRVPTGAT TQDAEVDDAKYGFVASGTLNPQKARVLLQL ALTQTKDPQQIQQIFNQY | *E. coli* L-asparaginase II precursor sequence |
| | | Escherichia coli str. K-12 substr. MG1655 |
| | | NCBI Reference Sequence: NP_417432.1 |
| | | Native paired cysteine residues |
| 11 | LPNITILATGGTIAGGGDSATKSNYTVGKV GVENLVNAVPQLKDIANVKGEQVVNIGSQD MNDNVWLTLAKKINTDCDKTDGFVITHGTD TMEETAYFLDLTVKCDKPVVMVGAMRPSTS MSADGPFNLYNAVVTAADKASANRGVLVVM NDTVLDGRDVTKTNTTDVATFKSVNYGPLG YIHNGKIDYQRTPARKHTSDTPFDVSKLNE LPKVGIVYNYANASDLPAKALVDAGYDGIV SAGVGNGNLYKSVFDTLATAAKTGTAVVRS SRVPTGATTQDAEVDDAKYGFVASGTLNPQ KARVLLQLALTQTKDPQQIQQIFNQY | *E. coli* L-asparaginase II mature sequence |
| 12 | LPNITILCTGGTIAGGGDSATKSNYTVGKV GCENLVNAVPQLKDIANVKGEQVVNIGSQD MNDNVWLTLAKKINTDCDKTDGFVITHGTD TMEETAYFLDLTVKCDKPVVMVGAMRPSTS MSADGPFNLYNAVVTAADKASANRGVLVVM NDTVLDGRDVTKTNTTDVATFKSVNYGPLG YIHNGKIDYQRTPARKHTSDTPFDVSKLNE LPKVGIVYNYANASDLPAKALVDAGYDGIV SAGVGNGNLYKSVFDTLATAAKTGTAVVRS SRVPTGATTQDAEVDDAKYGFVASGTLNPQ KARVLLQLALTQTKDPQQIQQIFNQY | Variant *E. coli* asparaginase mature sequence with one additional cysteine pair |
| | | A8C |
| | | V32C |
| 13 | MGKAVIAIHGGAGAISRAQMSLQQELRYIE ALSAIVETGQKMLEAGESALDVVTEAVRLL EECPLFNAGIGAVFTRDETHELDACVMDGN TLKAGAVAGVSHLRNPVLAARLVMEQSPHV MMIGEGAENFAFARGMERVSPEIFSTSLRY EQLLAARKEGATVLDHSGAPLDEKQKMGTV GAVALDLDGNLAAATSTGGMTNKLPGRVGD SPLVGAGCYANNASVAVSCTGTGEVFIRAL AAYDIAALMDYGGLSLAEACERVVMEKLPA LGGSGGLIAIDHEGNVALPFNTEGMYRAWG YAGDTPTTGIYREKGDTVATQ | *E. coli* L-asparaginase precursor sequence |
| | | [Isoaspartyl peptidase Escherichia coli str. K-12 substr. MG1655] |
| | | NCBI Reference Sequence: NP_415349.1 |
| | | paired cysteine residues |
| | | **3 unpaired cysteine residues** |
| 14 | QMSLQQELRYIEALSAIVETGQKMLEAGES ALDVVTEAVRLLEECPLFNAGIGAVFTRDE THELDACVMDGNTLKAGAVAGVSHLRNPVL AARLVMEQSPHVMMIGEGAENFAFARGMER VSPEIFSTSLRYEQLLAARKEGATVLDHSG APLDEKQKMGTVGAVALDLDGNLAAATSTG GMTNKLPGRVGDSPLVGAGCYANNASVAVS CTGTGEVFIRALAAYDIAALMDYGGLSLAE ACERVVMEKLPALGGSGGLIAIDHEGNVAL PFNTEGMYRAWGYAGDTPTTGIYREKGDTV ATQ | *E. coli* L-asparaginase mature sequence |
| | | paired cysteine residues |
| | | **3 unpaired cysteine residues** |
| 15 | QMSLQQELRYIEALSAIVETGQKMLEAGES ALDVVTEAVRLLEESPLFNAGIGAVFTRDE THELDACVMDGNTLKAGAVAGVSHLRNPVL AARLVMEQSPHVMMIGEGAENFAFARGMER VSPEIFSTSLRYEQLLAARKEGATVLDHSG APLDEKQKMGTVGAVALDLDGNLAAATSTG GMTNKLPGRVGDSPLVGAGCYANNASVAVS STGTGEVFIRALAAYDIAALMDYGGLSLAE ASERVVMEKLPALGGSGGLIAIDHEGNVAL PFNTEGMYRAWGYAGDTPTTGIYREKGDTV ATQ | Variant *E. coli* asparaginase mature sequence with no unpaired cysteines |
| | | C45S |
| | | C211S |
| | | C242S |

### DESCRIPTION OF THE EMBODIMENTS

Pegylation of biomolecules may prolong circulation time, protect against inactivation by proteolysis, mask immunogenic or antigenic sites, and/or reduce immunogenicity. ONCOSPAR^{®} (pegaspargase, Shire) is an FDA-approved pegylated asparaginase derived from *E*. *coli.* Based on the ONCASPAR^{®} package insert, each molecule of asparaginase has an average of 69-82 lysine-linked PEG molecules and is approximately 483-548 kDa. However, random pegylation can result in a heterogenous product and hypersensitivities to pegylated asparaginase derived from *E. coli* can still develop.

Asparaginase isolated from *E. chrysanthemi* has also been investigated. ERWINAZE^{®} (Jazz Pharmaceuticals), a native asparaginase derived from *E. chrysanthemi,* is FDA-approved as a substitute for pegaspargase and native *E. coli* asparaginase for patients who have developed hypersensitivity to *E*. coli-derived asparaginase. *See* ERWINAZE^{®} package insert; Gervais, D. Validation of a 30-year-old process for the manufacture of L-asparaginase from Erwinia chrysanthemi. Bioprocess Biosyst Eng (2013) 36:453-60. ERWINAZE^{®} has reportedly been in short supply. *See* fiercepharma.com/manufacturing/jazz-releases-some-erwinaze-children-s-leukemia-drug-has-been-shortage, dated February 26, 2018.

Pegylated recombinant *E. chrysanthemi* asparaginase was reported using random primary amine pegylation. The product had lower immunogenicity and improved efficacy. Chien, W., Pharmacology, immunogenicity, and efficacy of a novel pegylated recombinant Erwinia chrysanthemi-derived L-asparaginase. Invest New Drugs (2014) 32: 795-805. However, random pegylation may lead to a heterogenous product. Therefore, site-specific incorporation of PEG-conjugatable amino acids into bacterial asparaginases is advantageous, but not without issues.

Antigenic epitopes of *E. chrysanthemi* and *E. coli* asparaginases have been identified and mapped. *See* Moola, Z.B., et al. Epitope mapping and antigenic modification of L-asparaginase. Biochem J. (1994) 302: 921-927 and Werner A., et al. Mapping of B-cell epitopes in E. coli asparaginase II, an enzyme used in leukemia treatment. Biol. Chem. (2005) 386: 535-540. Nonetheless, the literature appears to be devoid of variant bacterial asparaginase products having site-specific, rather than random, pegylation for shielding immunogenic or antigenic sites (*e.g.*, via site-specific cysteine substitutions for thiol-specific conjugation). Recombinant expression of variant asparaginase polypeptides in the periplasm can be challenging. For example, while the oxidative environment of the periplasm is important for the formation of disulfide linkages between paired cysteine residues for proper protein folding, unpaired cysteine residues are also oxidized, hindering pegylation techniques.

Due to immunogenicity issues, shortages of clinical asparaginase products, and inefficient production methods, there is a need for asparaginase products having reduced immunogenicity, that are derived from alternative sources, and that are manufactured using more efficient production methods. Variant asparaginase polypeptides and methods that address these issues are described herein.

The present disclosure relates to variant asparaginase polypeptides having reduced immunogenicity and enhanced stability and pharmacokinetics, and methods of producing and using the same, for example, for treating acute lymphoblastic leukemia in humans and lymphoma in companion animals. For the convenience of the reader, the following definitions of terms used herein are provided.

As used herein, "a" or "an" means "at least one" or "one or more" unless otherwise specified. As used herein, the term "or" means "and/or" unless specified otherwise. In the context of a multiple dependent claim, the use of "or" when referring back to other claims refers to those claims in the alternative only.

### Exemplary asparaginase polypeptides

Variant asparaginase polypeptides are provided, for example, variant asparaginase polypeptides comprising PEG-conjugatable amino acid substitutions, such as cysteine and lysine substitutions for reduced immunogenicity.

"Amino acid sequence," means a sequence of amino acid residues in a peptide or protein. The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present disclosure, a "polypeptide" refers to a protein which includes modifications, such as deletions, additions, and substitutions (generally conservative in nature), to the native (or wildtype) sequence, as long as the protein maintains the desired activity. These modifications may be deliberate, as through site-directed mutagenesis, or may be accidental, such as through mutations of hosts which produce the proteins or errors due to PCR amplification.

An "amino acid derivative," as used herein, refers to any amino acid, modified amino acid, and/or amino acid analogue, that is not one of the 20 common natural amino acids found in humans. Exemplary amino acid derivatives include natural amino acids not found in humans (e.g., seleno cysteine and pyrrolysine, which may be found in some microorganisms) and unnatural amino acids. Exemplary amino acid derivatives, include, but are not limited to, amino acid derivatives commercially available through chemical product manufacturers and distributors (e.g., sigmaaldrich.com/chemistry/chemistry-products.html?TablePage=16274965, accessed on May 6, 2017, which is incorporated herein by reference). One or more amino acid derivative maybe incorporated into a polypeptide at a specific location using translation systems that utilize host cells, orthogonal aminoacyl-tRNA synthetases derived from eubacterial synthetases, orthogonal tRNAs, and an amino acid derivative. For further descriptions, see, e.g., U.S. Patent No.9,624,485.

"Asparaginase" or "L-asparaginase," as used herein is a polypeptide comprising the entirety or a fragment of asparaginase, including a variant polypeptide, a precursor polypeptide, a mature polypeptide, a tetramer, a dimer, a monomer, or any other form.

For example, "asparaginase" refers to an asparaginase polypeptide from any source, unless otherwise indicated, including plants, mammals (e.g., pigs and rodents), and bacteria (e.g., *E. coli*, *E. chrysanthemi*, *Aerobacter aerogenes*, *Aeromonas hydrophila*, *A. liquefaciens*, *A. salmonicida, A. sinuosa, Bacillus megaterium, B. subtilis, Erwinia amylovora, E. araoideae, E. carotovora, E. dissolvens, E. freundii, Hydrogenomonas eutropha, H. pantotropha, Photobacterium fischeri, Proteus americanus, P. mirabilis, P. morganii, P. paramericanus, P. psudovaleriei, P. shingides, P. vulgaris, Psudomonas acidovorans, P. ammoniagenes, P. asplenii, P. aureofaciens*, *P. caviae, P. convexa, P. dacunhae, P. fluorescens, Pgeniculata, P. lemonnieri, P. pavonacea, P. putida, P. reptilivora, Pseudomonas, P. stutzeri, P. synxantha, P. taetrolens, Serratia marcescens*, *Xanthomonas campestrils*, *etc*.; *See* Peterson RE and Ciegler A, L-asparaginase production by various bacteria. Applied Microbiol (1969) 17:929-930. In some embodiments, asparaginase comprises the amino acid sequence of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 14, or SEQ ID NO: 15.

"Wildtype" refers to a non-mutated version of a polypeptide that occurs in nature, or a fragment thereof. A wildtype polypeptide may be produced recombinantly or in nature. A wildtype polypeptide that is produced in nature is also referred to as a native polypeptide.

A "variant" means a biologically active polypeptide having at least about 80% amino acid sequence identity with the wildtype (or native) sequence polypeptide after aligning the sequences and introducing gaps, if necessary, to achieve the maximum percent sequence identity, and not considering any conservative substitutions as part of the sequence identity. Such variants include, for instance, polypeptides wherein one or more amino acid residues are added, deleted, at the N- or C-terminus of the polypeptide.

In some embodiments, a variant has at least about 80% amino acid sequence identity, at least about 85% amino acid sequence identity, at least about 90% amino acid sequence identity, at least about 95% amino acid sequence identity, at least about 97% amino acid sequence identity, at least about 98% amino acid sequence identity, or at least about 99% amino acid sequence identity with the wildtype (or native) sequence polypeptide.

A "biologically active" entity, or an entity having "biological activity," is an entity having any function related to or associated with a metabolic or physiological process, and/or having structural, regulatory, or biochemical functions of a naturally-occurring molecule. A biologically active polypeptide or fragment thereof includes one that can participate in a biological reaction, including, but not limited to, an enzymatic reaction, a ligand-receptor interaction or antigen-antibody binding. The biological activity can include an improved desired activity, or a decreased undesirable activity. An entity may demonstrate biological activity when it participates in a molecular interaction with another molecule, when it has therapeutic value in alleviating a disease condition, when it has prophylactic value in inducing an immune response, when it has diagnostic and/or prognostic value in determining the presence of a molecule.

As used herein, "percent (%) amino acid sequence identity" and "homology" with respect to a peptide, polypeptide, or antibody sequence are defined as the percentage of amino acid residues in a candidate sequence that are identical with the amino acid residues in the specific peptide or polypeptide sequence, after aligning the sequences and introducing gaps, if necessary to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, CLUSTAL OMEGA, ALIGN, or MEGALIGN^{™} (DNASTAR) software. Those skilled in the art can determine appropriate parameters for measuring alignment, including any parameters needed to achieve maximal alignment over the full length of sequences being compared.

As used herein, "site corresponding to amino acid position n" or "position corresponding to amino acid position n," wherein n is any number or range of numbers, refers to an amino acid position(s) of a subject polypeptide that aligns with position n of a reference polypeptide after aligning the amino acid sequence of the subject and reference polypeptides and introducing gaps. Alignment for purposes of whether a position of a subject polypeptide corresponds with position n of a reference polypeptide can be achieved in various ways that are within the skill in the art, for instance, using publicly available computer software such as BLAST, BLAST-2, CLUSTAL OMEGA, ALIGN, or MEGALIGN^{™} (DNASTAR) software. Those skilled in the art can determine appropriate parameters for alignment, including any parameters needed to achieve maximal alignment over the full length of two sequences being compared. In some embodiments, the subject polypeptide and the reference polypeptide are of different lengths.

An amino acid substitution may include but is not limited to the replacement of one amino acid in a polypeptide with another amino acid. Exemplary substitutions are shown in Table 2. Amino acid substitutions may be introduced into a protein of interest and the products screened for a desired activity, for example, retained/improved antigen binding or decreased immunogenicity.

**Table 2**

| Original Residue | Exemplary Substitutions |
|---|---|
| Ala (A) | Val; Leu; Ile |
| Arg (R) | Lys; Gln; Asn |
| Asn (N) | Gln; His; Asp; Lys; Arg |
| Asp (D) | Glu; Asn |
| Cys (C) | Ser; Ala |
| Gln (Q) | Asn; Glu |
| Glu (E) | Asp; Gln |
| Gly (G) | Ala |
| His (H) | Asn; Gln; Lys; Arg |
| Ile (I) | Leu; Val; Met; Ala; Phe; Norleucine |
| Leu (L) | Norleucine; Ile; Val; Met; Ala; Phe |
| Lys (K) | Arg; Gln; Asn |
| Met (M) | Leu; Phe; Ile |
| Phe (F) | Trp; Leu; Val; Ile; Ala; Tyr |
| Pro (P) | Ala |
| Ser (S) | Thr |
| Thr (T) | Val; Ser |
| Trp (W) | Tyr; Phe |
| Tyr (Y) | Trp; Phe; Thr; Ser |
| Val (V) | Ile; Leu; Met; Phe; Ala; Norleucine |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes with another class.

In some embodiments, a variant asparaginase polypeptide comprised the amino acid sequence of SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 12, and SEQ ID NO: 15.

A "PEG-conjugatable" amino acid refers to an amino acid or an amino acid derivative that is capable of associating with or covalently or non-covalently attaching to at least one PEG molecule.

In some embodiments, a variant asparaginase polypeptide comprises at least one PEG-conjugatable amino acid substitution. In some embodiments, a PEG-conjugatable amino acid comprises a lysine, a cysteine, a histidine, an arginine, an aspartic acid, a glutamine, a serine, a threonine, a tyrosine, or an amino acid derivative of any of those amino acids.

"PEG," as used herein, refers to a polyethylene glycol moiety.

"Pegylated," as used herein, refers to a polypeptide having one or more PEG moieties associated or covalently or non-covalently attached.

Sialylated," as used herein, refers to a polypeptide having one or more sialyic acid moieties covalently attached.

A variety of approaches for producing glycosylated and sialylated proteins have been developed. *See*, *e.g.*, Savinova, et al. Applied Biochem & Microbiol. (2015) 51(8): 827-833.

In some embodiments, a variant asparaginase polypeptide is pegylated and/or sialylated. In some embodiments, the variant asparaginase polypeptide is pegylated and/or sialylated at a cysteine, a lysine, a histidine, an arginine, an aspartic acid, a glutamine, a serine, a threonine, a tyrosine, or an amino acid derivative of any of those amino acids, such as an amino acid derivative of a cysteine, a lysine, a histidine, an arginine, an aspartic acid, a glutamine, a serine, a threonine, a tyrosine.

A "surface-exposed amino acid," refers to an amino acid residue having 30% or greater of its surface accessible to a solvent, as determined using any standard protein modeling software understood in the art, for example, Swiss-Model, MOE (Molecular Operating Environment), Rosetta, and Modeller.

In some embodiments, a surface-exposed amino acid includes an amino acid having 30% or greater surface exposure (or 30% or greater of its surface is accessible to a solvent), as determined by standard protein modeling software. In some embodiments, a surface-exposed amino acid includes an amino acid having 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, 95% or greater surface exposure.

A "partially embedded amino acid," refers to an amino acid residue having between 5% and 30% of its surface not accessible to a solvent, as determined using any standard protein modeling software understood in the art, for example, Swiss-Model, MOE (Molecular Operating Environment), Rosetta, and Modeller.

In some embodiments, a partially embedded amino acid includes an amino acid having between 5% and 30% surface exposure (or between 5% and 30% of its surface is accessible to a solvent), as determined by standard protein modeling software. In some embodiments, a surface-exposed amino acid includes an amino acid having between 5% and 25%, between 5% and 20%, between 5% and 15%, from 5% and 10%, between 10% and 20%, between 20% and 30%, or between 10% and 30% surface exposure.

"Immunogenic or antigenic site," as used herein refers to any amino acid or any group of amino acids within a polypeptide that is involved in eliciting an immune reaction.

In some embodiments, an immunogenic or antigenic site is any amino acid within an epitope. In some embodiments, an immunogenic or antigenic site is less immunogenic or less antigenic when the immunogenic or antigenic site, or an amino acid that is spatially near the immunogenic or antigenic site, is conjugated to PEG.

An amino acid that is "spatially near" another amino acid(s) refers to the three-dimensional proximity between them, as determined by three-dimensional protein modeling. An amino acid may be spatially near another amino acid if the distance between the two amino acids is 35 Angstroms (Å) or less, 30 Å or less, 25 Å or less, 20 Å or less, 15 Å or less, 10 Å or less, or 5 Å or less.

As used herein, the term "epitope" refers to a site on a target molecule (for example, an antigen, such as a protein, nucleic acid, carbohydrate or lipid) to which an antigen-binding molecule (for example, an antibody, antibody fragment, or scaffold protein containing antibody binding regions) binds. Epitopes often include a chemically active surface grouping of molecules such as amino acids, polypeptides or sugar side chains and have specific three-dimensional structural characteristics as well as specific charge characteristics. Epitopes can be formed both from contiguous or juxtaposed noncontiguous residues (for example, amino acids, nucleotides, sugars, lipid moiety) of the target molecule. Epitopes formed from contiguous residues (for example, amino acids, nucleotides, sugars, lipid moiety) typically are retained on exposure to denaturing solvents whereas epitopes formed by tertiary folding typically are lost on treatment with denaturing solvents. An epitope may include but is not limited to at least 3, at least 5 or 8-10 residues (for example, amino acids or nucleotides). In some examples an epitope is less than 20 residues (for example, amino acids or nucleotides) in length, less than 15 residues or less than 12 residues. Two antibodies may bind the same epitope within an antigen if they exhibit competitive binding for the antigen. In some embodiments, an epitope can be identified by a certain minimal distance to a CDR residue on the antigen-binding molecule. In some embodiments, an epitope can be identified by the above distance, and further limited to those residues involved in a bond (for example, a hydrogen bond) between an antibody residue and an antigen residue. An epitope can be identified by various scans as well, for example an alanine or arginine scan can indicate one or more residues that the antigen-binding molecule can interact with. Unless explicitly denoted, a set of residues as an epitope does not exclude other residues from being part of the epitope for a particular antibody. Rather, the presence of such a set designates a minimal series (or set of species) of epitopes. Thus, in some embodiments, a set of residues identified as an epitope designates a minimal epitope of relevance for the antigen, rather than an exclusive list of residues for an epitope on an antigen.

In some embodiments, an immunogenic or antigenic site may be determined by epitope-scanning methods understood in the art. For example, according to the methods described in Moola, ZB, Erwinia chrysanthemi L-asparaginase: epitope mapping and production of antigenically modified enzymes. Biochem J. (1994) 921-927.

In some embodiments, an immunogenic or antigenic site is an epitope corresponding to amino acid positions 37 to 40 or 205 to 212 of SEQ ID NO: 2. In some embodiments, an immunogenic or antigenic site corresponds to amino acid position 37, 38, 39, 40, 41, 72, 205, 206, 207, 208, 209, 210, 211, 212, 265, or 288 of SEQ ID NO: 2. In some embodiments, an immunogenic or antigenic site corresponds to amino acid position 55, 56, 57, 58, 114, 115, 116, 117, 118, 119, 201, 202, 203, 204, 205, 206, 207, 252, 253, 254, 255, 256, 257, or 258 of SEQ ID NO: 11.

In some embodiments, a variant asparaginase polypeptide comprises at least one PEG-conjugatable amino acid substitution or at least one cysteine substitution at at least one position corresponding to a position selected from position 3, 4, 17, 26, 37, 38, 41, 42, 44, 45, 47, 48, 51, 53, 54, 55, 56, 59, 60, 68, 72, 79, 82, 83, 84, 85, 87, 110, 112, 123, 124, 125, 127, 180, 190, 191, 192, 198, 202, 205, 206, 208, 210, 212, 213, 215, 216, 219, 231, 239, 240, 241, 243, 257, 260, 261, 264, 265, 267, 268, 269, 270, 280, 286, 287, 288, 289, 312, 313, 315, 316, 317, 318, and 322 of SEQ ID NO: 2.

In some embodiments, a variant asparaginase polypeptide comprises at least one PEG-conjugatable amino acid substitution or at least one lysine substitution at at least one position corresponding to a position selected from position 4, 17, 26, 37, 38, 41, 42, 44, 45, 51, 53, 54, 55, 56, 59, 60, 68, 79, 82, 83, 84, 85, 87, 112, 123, 124, 125, 127, 180, 190, 191, 192, 198, 202, 205, 206, 208, 210, 212, 213, 215, 216, 231, 239, 240, 241, 257, 260, 261, 264, 267, 268, 270, 280, 286, 287, 288, 289, 312, 313, 315, 316, 317, and 322 of SEQ ID NO: 2.

In some embodiments, a variant asparaginase polypeptide comprises at least one cysteine located at at least one position corresponding to a position selected from 51, 52, 118, 119, 196, 206, 285, and 311 of SEQ ID NO: 11. In some embodiments, a variant asparaginase polypeptide comprises at least one pair of cysteines selected from any one or combination of C1-C2 paired cysteine substitution(s) listed in Table 5. For example, a variant asparaginase polypeptide may comprise one or more pairs of cysteines located at a position corresponding to amino acid positions 116 and 120, amino acid positions 196 and 200, or amino acid positions 225 and 252 of SEQ ID NO: 11.

The term "affinity" means the strength of the sum total of noncovalent interactions between a single binding site of a molecule (for example, an antibody) and its binding partner (for example, an antigen). The affinity of a molecule X for its partner Y can generally be represented by the dissociation constant (K_{D}). Affinity can be measured by common methods known in the art, such as, for example, immunoblot, ELISA KD, KinEx A, biolayer interferometry (BLI), or surface plasmon resonance devices.

The terms "K_{D}," "K_{d}," "Kd" or "Kd value" as used interchangeably to refer to the equilibrium dissociation constant of an antibody-antigen interaction. In some embodiments, the K_{d} of the antibody is measured by using biolayer interferometry assays using a biosensor, such as an Octet^{®} System (Pall ForteBio LLC, Fremont, CA) according to the supplier's instructions. Briefly, biotinylated antigen is bound to the sensor tip and the association of antibody is monitored for ninety seconds and the dissociation is monitored for 600 seconds. The buffer for dilutions and binding steps is 20 mM phosphate, 150 mM NaCl, pH 7.2. A buffer only blank curve is subtracted to correct for any drift. The data are fit to a 2:1 binding model using ForteBio data analysis software to determine association rate constant (kₒₙ), dissociation rate constant (k_{off}), and the K_{d}. The equilibrium dissociation constant (K_{d}) is calculated as the ratio of k_{off}/kₒₙ. The term "kon" refers to the rate constant for association of an antibody to an antigen and the term "koff" refers to the rate constant for dissociation of an antibody from the antibody/antigen complex.

"Surface plasmon resonance" denotes an optical phenomenon that allows for the analysis of real-time biospecific interactions by detection of alterations in protein concentrations within a biosensor matrix, for example using the BIAcore^{™} system (BIAcore International AB, a GE Healthcare company, Uppsala, Sweden and Piscataway, N.J.). For further descriptions, see Jonsson et al. (1993) Ann. Biol. Clin. 51: 19-26.

"Biolayer interferometry" refers to an optical analytical technique that analyzes the interference pattern of light reflected from a layer of immobilized protein on a biosensor tip and an internal reference layer. Changes in the number of molecules bound to the biosensor tip cause shifts in the interference pattern that can be measured in real-time. A nonlimiting exemplary device for biolayer interferometry is an Octet^{®} system (Pall ForteBio LLC). See, e.g., Abdiche et al., 2008, Anal. Biochem. 377: 209-277.

To "reduce" or "inhibit" means to decrease, reduce, or arrest an activity, function, or amount as compared to a reference. In some embodiments, by "reduce" or "inhibit" is meant the ability to cause an overall decrease of 20% or greater. In some embodiments, by "reduce" or "inhibit" is meant the ability to cause an overall decrease of 50% or greater. In some embodiments, by "reduce" or "inhibit" is meant the ability to cause an overall decrease of 75%, 85%, 90%, 95%, or greater. In some embodiments, the amount noted above is inhibited or decreased over a period of time, relative to a control dose (such as a placebo) over the same period of time. A "reference" as used herein, refers to any sample, standard, or level that is used for comparison purposes. A reference may be obtained from a healthy or non-diseased sample. In some examples, a reference is obtained from a non-diseased or non-treated sample of a companion animal. In some examples, a reference is obtained from one or more healthy animals of a particular species, which are not the animal being tested or treated.

The term "substantially reduced," as used herein, denotes a sufficiently high degree of reduction between a numeric value and a reference numeric value such that one of skill in the art would consider the difference between the two values to be of statistical significance within the context of the biological characteristic measured by said values. In some embodiments, the substantially reduced numeric values is reduced by greater than about any one of 10%, 15% 20%, 25%, 30%, 35%, 40%, 45%, 50%, 60%, 70%, 80%, 90%, or 100% compared to the reference value.

### Exemplary expression and production

Polynucleotide sequences that encode all or part of an asparaginase polypeptide with or without a leader sequence are provided. If a homologous leader sequence (i.e., a leader sequence of a wildtype asparaginase) is not used in the construction of the nucleic acid molecule, then another bacterial leader sequence may be used.

Typically, a nucleotide sequence encoding the polypeptide of interest, such as an asparaginase polypeptide described herein, is inserted into an expression vector, suitable for expression in a selected host cell.

The term "vector" is used to describe a polynucleotide that can be engineered to contain a cloned polynucleotide or polynucleotides that can be propagated in a host cell. A vector can include one or more of the following elements: an origin of replication, one or more regulatory sequences (such as, for example, promoters or enhancers) that regulate the expression of the polypeptide of interest, or one or more selectable marker genes (such as, for example, antibiotic resistance genes and genes that can be used in colorimetric assays, for example, β-galactosidase). The term "expression vector" refers to a vector that is used to express a polypeptide of interest in a host cell.

A "host cell" refers to a cell that may be or has been a recipient of a vector or isolated polynucleotide. Host cells may be prokaryotic cells or eukaryotic cells. Exemplary prokaryotic cells include *E. coli*, *bacillus*, and *pseudomonas.* Exemplary eukaryotic cells include mammalian cells, such as primate or non-primate animal cells; fungal cells, such as yeast; plant cells; and insect cells. Nonlimiting exemplary mammalian cells include, but are not limited to, NS0 cells, PER.C6^{®} cells (Crucell), 293 cells, and CHO cells, and their derivatives, such as 293-6E, DG44, CHO-S, and CHO-K cells. Host cells include progeny of a single host cell, and the progeny may not necessarily be completely identical (in morphology or in genomic DNA complement) to the original parent cell due to natural, accidental, or deliberate mutation. A host cell includes cells transfected in vivo with a polynucleotide(s) encoding an amino acid sequence(s) provided herein.

The term "isolated" as used herein refers to a molecule that has been separated from at least some of the components with which it is typically found in nature or produced. For example, a polypeptide is referred to as "isolated" when it is separated from at least some of the components of the cell in which it was produced. Where a polypeptide is secreted by a cell after expression, physically separating the supernatant containing the polypeptide from the cell that produced it is considered to be "isolating" the polypeptide. Similarly, a polynucleotide is referred to as "isolated" when it is not part of the larger polynucleotide (such as, for example, genomic DNA or mitochondrial DNA, in the case of a DNA polynucleotide) in which it is typically found in nature, or is separated from at least some of the components of the cell in which it was produced, for example, in the case of an RNA polynucleotide. Thus, a DNA polynucleotide that is contained in a vector inside a host cell may be referred to as "isolated." In some embodiments, the asparaginase is purified using chromatography, such as size exclusion chromatography, ion exchange chromatography, hydrophobic interaction chromatography, and CHT chromatography or mixed mode chromatography.

### Exemplary Pharmaceutical Compositions

The terms "pharmaceutical formulation" and "pharmaceutical composition" refer to a preparation which is in such form as to permit the biological activity of the active ingredient(s) to be effective, and which contains no additional components that are unacceptably toxic to a subject to which the formulation would be administered.

A "pharmaceutically acceptable carrier" refers to a non-toxic solid, semisolid, or liquid filler, diluent, encapsulating material, formulation auxiliary, or carrier conventional in the art for use with a therapeutic agent that together comprise a "pharmaceutical composition" for administration to a subject. A pharmaceutically acceptable carrier is non-toxic to recipients at the dosages and concentrations employed and is compatible with other ingredients of the formulation. The pharmaceutically acceptable carrier is appropriate for the formulation employed. Examples of pharmaceutically acceptable carriers include alumina; aluminum stearate; lecithin; serum proteins, such as human serum albumin, canine or other animal albumin; buffers such as phosphate, citrate, tromethamine or HEPES buffers; glycine; sorbic acid; potassium sorbate; partial glyceride mixtures of saturated vegetable fatty acids; water; salts or electrolytes, such as protamine sulfate, disodium hydrogen phosphate, potassium hydrogen phosphate, sodium chloride, zinc salts, colloidal silica, or magnesium trisilicate; polyvinyl pyrrolidone, cellulose-based substances; polyethylene glycol; sucrose; mannitol; or amino acids including, but not limited to, arginine.

The pharmaceutical composition can be stored in lyophilized form. Thus, in some embodiments, the preparation process includes a lyophilization step. The lyophilized composition may then be reformulated, typically as an aqueous composition suitable for parenteral administration, prior to administration to the subject. In other embodiments, particularly where the polypeptide is highly stable to thermal and oxidative denaturation, the pharmaceutical composition can be stored as a liquid, i.e., as an aqueous composition, which may be administered directly, or with appropriate dilution, to the subject. A lyophilized composition can be reconstituted with sterile Water for Injection (WFI). Bacteriostatic reagents, such benzyl alcohol, may be included. Thus, the invention provides pharmaceutical compositions in solid or liquid form.

The pH of the pharmaceutical compositions may be in the range of from about pH 5 to about pH 8, when administered. The compositions of the invention are sterile if they are to be used for therapeutic purposes. Sterility can be achieved by any of several means known in the art, including by filtration through sterile filtration membranes (e.g., 0.2 micron membranes). Sterility may be maintained with or without anti-bacterial agents.

### Exemplary uses of variant asparaginase polypeptides

The variant asparaginase polypeptides or pharmaceutical compositions comprising the variant asparaginase polypeptides of the invention may be useful for treating a variety of disease conditions, including cancers such as lymphomas or leukemias. In some embodiments, the variant asparaginase polypeptides or pharmaceutical compositions comprising the variant asparaginase polypeptides of the invention may be used in the treatment of ALL in humans or lymphomas in companion animal species.

The term "companion animal species" refers to an animal suitable to be a companion to humans. In some embodiments, a companion animal species is a small mammal, such as a canine, feline, dog, cat, horse, rabbit, ferret, guinea pig, rodent, etc. In some embodiments, a companion animal species is a farm animal, such as a horse, cow, pig, etc.

As used herein, "treatment" is an approach for obtaining beneficial or desired clinical results. "Treatment" as used herein, covers any administration or application of a therapeutic for disease in a mammal, including a companion animal. For purposes of this disclosure, beneficial or desired clinical results include, but are not limited to, any one or more of: alleviation of one or more symptoms, diminishment of extent of disease, preventing or delaying spread of disease, preventing or delaying recurrence of disease, delay or slowing of disease progression, amelioration of the disease state, inhibiting the disease or progression of the disease, inhibiting or slowing the disease or its progression, arresting its development, and remission (whether partial or total). Also encompassed by "treatment" is a reduction of pathological consequence of a proliferative disease. The methods provided herein contemplate any one or more of these aspects of treatment. In-line with the above, the term treatment does not require one-hundred percent removal of all aspects of the disorder.

A "therapeutically effective amount" of a substance/molecule, agonist or antagonist may vary according to factors such as the type of disease to be treated, the disease state, the severity and course of the disease, the type of therapeutic purpose, any previous therapy, the clinical history, the response to prior treatment, the discretion of the attending veterinarian, age, sex, and weight of the animal, and the ability of the substance/molecule, agonist or antagonist to elicit a desired response in the animal. A therapeutically effective amount is also one in which any toxic or detrimental effects of the substance/molecule, agonist or antagonist are outweighed by the therapeutically beneficial effects. A therapeutically effective amount may be delivered in one or more administrations. A therapeutically effective amount refers to an amount effective, at dosages and for periods of time necessary, to achieve the desired therapeutic or prophylactic result.

In some embodiments, a variant asparaginase polypeptides or pharmaceutical composition comprising a variant asparaginase polypeptide, as described herein, is administered parenterally, by subcutaneous administration, intravenous infusion, or intramuscular injection. In some embodiments, a variant asparaginase polypeptide or pharmaceutical composition is administered as a bolus injection or by continuous infusion over a period of time. In some embodiments, a variant asparaginase polypeptide or pharmaceutical composition is administered by an intramuscular, an intraperitoneal, an intracerebrospinal, a subcutaneous, an intra-arterial, an intrasynovial, an intrathecal, or an inhalation route.

In some embodiments, the dose is administered once per week for at least two or three consecutive weeks, and in some embodiments, this cycle of treatment is repeated two or more times, optionally interspersed with one or more weeks of no treatment. In other embodiments, the therapeutically effective dose is administered once per day for two to five consecutive days, and in some embodiments, this cycle of treatment is repeated two or more times, optionally interspersed with one or more days or weeks of no treatment.

Administration "in combination with" one or more further therapeutic agents includes simultaneous (concurrent) and consecutive or sequential administration in any order. The term "concurrently" is used herein to refer to administration of two or more therapeutic agents, where at least part of the administration overlaps in time or where the administration of one therapeutic agent falls within a short period of time relative to administration of the other therapeutic agent. For example, the two or more therapeutic agents are administered with a time separation of no more than about a specified number of minutes. The term "sequentially" is used herein to refer to administration of two or more therapeutic agents where the administration of one or more agent(s) continues after discontinuing the administration of one or more other agent(s), or wherein administration of one or more agent(s) begins before the administration of one or more other agent(s). For example, administration of the two or more therapeutic agents are administered with a time separation of more than about a specified number of minutes. As used herein, "in conjunction with" refers to administration of one treatment modality in addition to another treatment modality. As such, "in conjunction with" refers to administration of one treatment modality before, during or after administration of the other treatment modality to the animal.

The following examples illustrate particular aspects of the disclosure and are not intended in any way to limit the disclosure.

### EXAMPLES

### Example 1

### E. chrysanthemi asparaginase expression in E. coli

Native *E. chrysanthemi* asparaginase expressed in its native host cell is typically processed and secreted into the periplasmic space or media. Expression of *E. chrysanthemi* asparaginase in *E. coli* cells was investigated. The nucleotide sequence encoding native *E*. *chrysanthemi* asparaginase containing a leader sequence (SEQ ID NO: 1) was cloned between the Xba1 and HindIII sites of pET28a(+) (Novagen), an *E. coli* expression vector having a T7 promoter and kanamycin resistance gene. After transforming the resulting plasmid into *E. coli* BL21(DE3) cells, the cells were incubated at 37 °C until the optical density at 600 nm reached approximately 1. 1 mM IPTG was then added to the culture medium and the cells incubated at 37 °C for 2 hours to induce expression of the *E. chrysanthemi* asparaginase protein.

The cells were subjected to osmotic shock to release proteins from the periplasm. The cells were centrifuged and the supernatant (also called the osmotic fraction) was isolated. The cell pellet was resuspended and subjected to sonication to lyse the cells. The cells were centrifuged and the lysate (also called the soluble fraction) was separated from the pellet (also called the insoluble fraction). The three fractions (osmotic, soluble, and insoluble fractions) were separated by SDS-PAGE and visualized using Coomassie stain.

Based on band intensity and difference in molecular weight, the osmotic fraction appeared to contain very low yields of processed asparaginase, while a large majority of the full length asparaginase product was observed in the cell pellet (insoluble fraction). A low amount of unprocessed asparagine product was observed in the lysate fraction (soluble fraction). These results suggest that periplasmic expression of *E. chrysanthemi* asparaginase in *E. coli* was inefficient and the bulk of the unprocessed asparaginase product was trapped inside the cells in an insoluble form (e.g., aggregates, inclusion bodies, etc.), possibly due to failed processing of the leader sequence.

### Example 2

### Intracellular expression of E. chrysanthemi asparaginase

Expression of *E. chrysanthemi* asparaginase lacking a leader sequence in *E. coli* cells was also investigated. The nucleotide sequence encoding native *E. chrysanthemi* asparaginase lacking its leader sequence (SEQ ID NO: 3) was cloned between the Xba1 and HindIII sites of pET28a(+) (Novagen), transformed into *E*. *coli* BL21(DE3) cells, and cultured in the presence of 1 mM IPTG as described in Example 1. The cells were lysed by sonication, centrifuged, and the lysate (soluble fraction) and pellet (insoluble fraction) were separated by SDS-PAGE and visualized using Coomassie stain. Surprisingly, a higher yield of soluble, intracellularly expressed asparaginase was observed in the lysate.

### Example 3

### Variant E. chrysanthemi asparaginase polypeptides for site-directed thiol conjugation

Secreted proteins of gram-negative bacteria typically contain disulfide(s) linkages that are catalyzed at cysteine residues by thiol-disulfide oxidoreductases in the periplasm. For example, native *E. coli* asparaginase has one disulfide linkage between the proteins' only two cysteine residues. However, inspection of the primary amino acid sequence of *E. chrysanthemi* asparaginase revealed that the protein lacks cysteine residues. This observation suggests that native *E. chrysanthemi* asparaginase lacks disulfide linkages and that the oxidative environment of the periplasmic space may be not needed for folding of this protein.

Bacterial proteins are highly immunogenic to humans. Pegylation of bacterial proteins can shield the immunogenic locations and reduce immunogenicity. In addition, pegylation may extend the in vivo half-life of the protein. For ONCASPAR^{®} (pegasparagase, Shire), the *E*. coli-derived asparaginase is pegylated at surface-exposed primary amines (e.g., lysine residues). Unfortunately, such non-specific pegylation may lead to a heterogenous product. Pegylation at specific immunogenic sites rather than across the entire protein may be advantageous.

Since native *E. chrysanthemi* asparaginase does not include cysteine residues, variants comprising one or more cysteine substitution(s) may be specifically pegylated at those locations. The introduction of one or more cysteine residue(s) at or near immunogenic or antigenic sites of *E. chrysanthemi* asparaginase was investigated. Three-dimensional protein structure analysis of tetrameric *E. chrysanthemi* asparaginase was used to identify locations of surface-exposed amino acids at or spatially near potential immunogenic or antigenic sites at which pegylation may shield the site without affecting its active site(s). Potential immunogenic or antigenic sites include amino acid positions 37 to 41, 72, 205 to 212, 265, and 288 of the mature amino acid sequence (SEQ ID NO: 2). Table 2 lists amino acid locations of SEQ ID NO: 2 at which a cysteine residue may be introduced for site-specific pegylation for reduced immunogenicity.

**Table 2.**

| Cysteine substitution(s) for pegylation of *E*. *chrysanthemi* asparaginase (based on mature amino acid sequence SEQ ID NO: 2) | | | |
|---|---|---|---|
| K3C | D68C | L202C | R264C |
| L4C | K72C | T205C | K265C |
| A17C | E79C | R206C | L267C |
| T26C | A82C | V208C | E268C |
| D37C | R83C | D210C | K269C |
| T38C | D84C | R212C | G270C |
| N41C | D85C | G213C | G280C |
| A42C | D87C | T215C | P286C |
| P44C | K110C | S216C | D287C |
| E45C | D112C | K219C | E288C |
| K47C | P123C | E231C | E289C |
| K48C | A124C | Q239C | T312C |
| N51C | T125C | H240C | R313C |
| K53C | I127C | G241C | S315C |
| G54C | N180C | K243C | D316C |
| E55C | G190C | V257C | P317C |
| Q56C | N191C | 1260C | K318C |
| N59C | R192C | A261C | E322C |
| M60C | R198C | | |

Variant *E. chrysanthemi* asparaginase polypeptides may be prepared by introducing one or more cysteine residues at any one or any combination of amino acid location(s) listed in Table 2. For example, 2, 3, 4, 5, 6, or more cysteine residues may be introduced into *E. chrysanthemi* asparaginase.

By way of example, variant precursor *E. chrysanthemi* asparaginase polypeptides having three cysteine amino acid substitutions at positions 41, 72, and 265 (SEQ ID NO: 4) or having four cysteine amino acid substitutions at positions 41, 72, 265, and 288 (SEQ ID NO: 7) of the mature amino acid sequence may be prepared.

### Example 4

### Intracellular expression of variant E. chrysanthemi asparaginase polypeptides

While the oxidative environment of the periplasm catalyzes disulfide linkage between cysteine residues, cysteine thiol groups are protected from oxidation in the reducing environment of the cytoplasm. The intracellular expression of a variant *E. chrysanthemi* asparaginase polypeptide comprising cysteine substitutions at immunogenic or antigenic sites was explored in *E. coli* cells. The nucleotide sequence encoding a variant *E*. *chrysanthemi* asparaginase having an N-terminal poly-His tag in place of the leader sequence and having three cysteine amino acid substitutions at positions 41, 72, and 265 of the mature amino acid sequence (SEQ ID NO: 5) was cloned into pET28a(+) (Novagen). After transforming the resulting plasmid into *E. coli* BL21(DE3) cells, the cells were incubated at 37 °C until the optical density at 600nm reached approximately 1. 1 mM IPTG was then added to the culture medium and the cells incubated at 37 °C for 2 hours to induce expression of the variant *E. chrysanthemi* asparaginase protein.

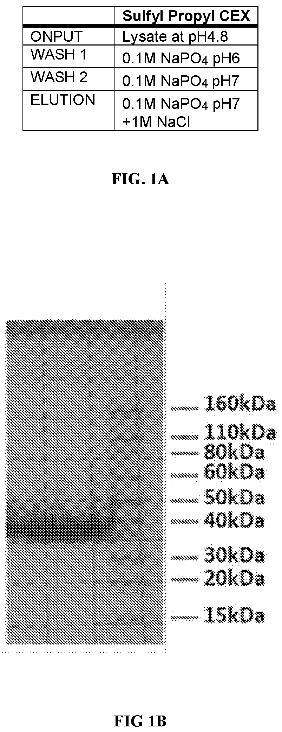

The cells were lysed by sonication, centrifuged, and the variant *E. chrysanthemi* asparaginase (SEQ ID NO: 5) purified from the lysate by affinity chromatography using Ni Sepharose excel histine-tagged protein purification resin (GE Healthcare, catalog number 17371201) followed by cation exchange chromatography using Sulfphopropyl Sepharose Fast Flow (SP FF) cation exchange chromatography resin (GE Healthcare, catalog number 17072901). The SP FF column was washed with 0.1 M sodium phosphate at pH 6 followed by pH 7, and the protein was eluted with a solution of 0.1 M sodium phosphate (pH 7) and 1 M sodium chloride. See FIG. 1A. The purified protein was separated using non-reducing SDS-PAGE and visualized using Coomassie staining (FIG. 1B).

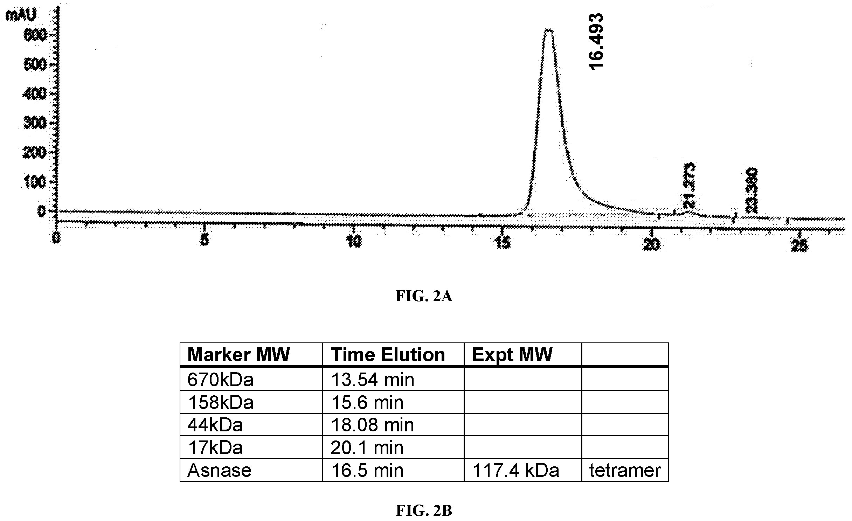

In addition, size exclusion chromatography was used to compare the hydrodynamic radius of the purified, variant *E. chrysanthemi* asparaginase (SEQ ID NO: 5) to that of Novex Sharp Prestained Protein Standard markers (ThermoFisher, catalog number LC5800), and to confirm that the purified protein was in tetrameric form. The purified protein was loaded onto a Shodex KW803 column (8 mm x 300 mm) with a KW-G guard column using Agilent's 1100 chromatography system at a constant flow rate of 0.5 mL per minute and a running buffer of 0.1 M sodium phosphate pH 6.6 and 1 M sodium chloride. The eluted material was monitored by absorbance at a wavelength of 214 nm. The purified, variant *E*. *chrysanthemi* asparaginase product (SEQ ID NO: 5) eluted at 16.5 minutes, consistent with the predicted molecular weight for the tetrameric form of the protein in solution (~117.4 kDa). See FIG. 2A and FIG. 2B.

### Example 5

### Site-directed pegylation of variant E. chrysanthemi asparaginase polypeptides

Conjugation of purified, variant *E. chrysanthemi* asparaginase (SEQ ID NO: 5) was performed using (1) random amine pegylation with α-succinimidyloxyglutaryl-ω-methoxy, polyoxyethylene (m.w. 5 kD; SUNBRIGHT^{®} ME-050GS; NOF Corporation), (2) thiol-specific pegylation with α-[3-(3-Maleimido-1-oxopropyl)amino]propyl-ω-methoxy, polyoxyethylene (m.w. 10 kD; SUNBRIGHT^{®} ME-100MA; NOF Corporation), or (3) both methods, according to the manufacturer's instructions.

For the thiol-specific pegylation, different reaction times (5 and 60 minutes) and different reaction temperatures (room temperature and 37° C) were tested. The pegylated proteins were separated by SDS-PAGE under reducing (with +DTT) and non-reducing (without DTT) conditions and visualized using Coomassie staining (FIG. 3). The unpegylated protein ran at about 35 kDa under reducing (FIG. 3, lane 2) and nonreducing conditions (FIG. 3, lane 3), suggesting that no disulfide bonds were present in purified, variant *E. chrysanthemi* asparaginase (SEQ ID NO: 5). The pegylated samples (FIG. 3, lanes 4-9) ran at a higher molecular weight consistent with pegylation of the protein.

Asparaginase activity of variant *E. chrysanthemi* asparaginase (SEQ ID NO: 5) conjugated via random amine pegylation and/or thiol-specific pegylation, as described above, was measured using an Asparaginase Activity assay kit (BioVision; catalog number K754-100) in duplicate according to the manufacturer's instructions. The results of the assay are provided in Table 3, below. The results show that variant *E. chrysanthemi* asparaginase (SEQ ID NO: 5) maintained enzymatic activity after pegylation.

**Table 3**

| **Sample** | **Type of asparaginase** | **Pegylation** | **Avg RFU*** | **Activity (nmol)** | **Specific Activity (nmol/µg)** |
|---|---|---|---|---|---|
| A | SEQ ID NO: 6 | none | 4303* | | |
| B | E. coli asparaginase from assay kit | none | 5659 | 24 | 24 |
| C | E. coli asparaginase from assay kit | amine | 2616 | 7 | 7 |
| D | SEQ ID NO: 6 | none | 3276 | 11 | 9 |
| E | SEQ ID NO: 6 | amine | 3643 | 13 | 10 |
| F | SEQ ID NO: 5 | none | 4066.5 | 15 | 50 |
| G | SEQ ID NO: 5 | amine | 4570 | 18 | 60 |
| H | SEQ ID NO: 5 | thiol | 4746.5 | 19 | 63 |
| I | SEQ ID NO: 5 | amine & thiol | 4094 | 15 | 50 |

| | | | | | |
|---|---|---|---|---|---|
| * Each assay was run in duplicate, except sample A was run once. | | | | | |

### Example 6

### Variant E. chrysanthemi asparaginase polypeptides for site-directed amine conjugation

Surface exposed primary amines (e.g., lysine) can be conjugated to amine-reactive PEG derivatives. The pegylation is likely to be less specific and the product may be heterogenous. Introducing one or more additional lysine residue(s) at surface-exposed locations can provide additional sites for amine-reactive pegylation. For example, variant *E. chrysanthemi* asparaginase polypeptides may be prepared by substituting one or more lysine residues at or near potential immunogenic or antigenic sites, such as amino acid positions 41, 72, 265, and 288 of the mature amino acid sequence (SEQ ID NO: 2). Table 4 lists exemplary surface-exposed amino acid locations of *E. chrysanthemi* asparaginase at or spatially near amino acid positions 41, 72, 265, and 288 of the mature amino acid sequence (SEQ ID NO: 2), at which a lysine residue may be introduced for site-specific pegylation.

**Table 4.**

| Lysine substitution(s) for pegylation of *E*. *chrysanthemi* asparaginase (based on mature amino acid sequence SEQ ID NO: 2) | | | |
|---|---|---|---|
| L4K | E79K | L202K | R264K |
| A17K | A82K | T205K | L267K |
| T26K | R83K | R206K | E268K |
| D37K | D84K | V208K | G270K |
| T38K | D85K | D210K | G280K |
| N41K | D87K | R212K | P286K |
| A42K | D112K | G213K | D287K |
| P44K | P123K | T215K | E288K |
| E45K | A124K | S216K | E289K |
| N51K | T125K | E231K | T312K |
| G54K | I127K | Q239K | R313K |
| E55K | N180K | H240K | S315K |
| Q56K | G190K | G241K | D316K |
| N59K | N191K | V257K | P317K |
| M60K | R192K | 1260K | E322K |
| D68K | R198K | A261K | |

Variant *E. chrysanthemi* asparaginase polypeptides may be prepared by introducing one or more lysine residues at any one or any combination of amino acid location(s) listed in Table 4. For example, 2, 3, 4, 5, 6, or more lysine residues may be introduced into *E. chrysanthemi* asparaginase.

By way of example, variant precursor *E. chrysanthemi* asparaginase polypeptides having a lysine amino acid substitution at position 41 (SEQ ID NO: 8) or at positions 41 and 288 (SEQ ID NO: 9) of the mature amino acid sequence may be prepared.

### Example 7

### Variant E. coli asparaginase polypeptides for site-directed amine conjugation

*E. coli* L-asparaginase II (SEQ ID NO: 10) is currently approved for the treatment of acute lymphoblastic leukemia. Unfortunately, immunological reactions are a significant adverse side effect. Site-specific pegylation would be advantageous to reduce immunological side effects. The native *E. coli* asparaginase protein has a leader sequence and two cysteine residues, which form a single disulfide linkage. It is thought that the protein is processed and secreted into the periplasmic space to form the disulfide linkage. Unlike *E. chrysanthemi* asparaginase, *E. coli* asparaginase may not be suitable for intracellular expression because disulfide linkage in the periplasm may be necessary for proper protein folding. Different approaches for site-specific pegylation of *E. coli* asparaginase were investigated.

One approach for introducing specific pegylation sites into *E. coli* asparaginase involves substituting unpaired cysteines at locations that are partially embedded at or near potential immunogenic or antigenic sites. The thiol group of unpaired cysteines at partially embedded sites may be shielded from oxidation in the periplasm, yet be sufficiently exposed for conjugation reactions, such as pegylation. Three-dimensional protein structure analysis of tetrameric *E. coli* L-Asparaginase II was used to identify locations of partially embedded amino acids at or spatially near potential immunogenic or antigenic sites at which pegylation may shield the site without affecting its active site(s). For example, one or more unpaired cysteine residues may be substituted at partially embedded sites at or spatially near immunogenic or antigenic sites, such as amino acid positions 55-58, 114-119, 201-207, and 252-258 of the mature amino acid sequence (SEQ ID NO: 11). By way of example, variant *E. coli* asparaginase polypeptides may be prepared by substituting one or more unpaired cysteine residues at any one or more of the following amino acid locations based on the mature amino acid sequence (SEQ ID NO: 11): E51C, Q52C, S118C, T119C, K196C, S206C, D285C, and T311C.

A second method for introducing specific pegylation sites into *E. coli* asparaginase involves introducing one or more paired cysteine residues at or spatially near potential immunogenic or antigenic sites. Rather than the thiol group of the substituted cysteine pairs being oxidized in the periplasm, the pair(s) should form disulfide linkage(s). The disulfide linkages (including the original linkage) may then be reduced using a reducing agent, such as DTT, to allow for conjugation. Pegylation or labeling may be accomplished by rebridging the paired cysteines using various chemistries, such as the thiol-yne coupling reaction. *See* Griebenow N., et al., Site-specific conjugation of peptides and proteins via rebridging of disulfide bonds using the thiol-yne coupling reaction. Bioconjug Chem (2016) 4: 911-917.

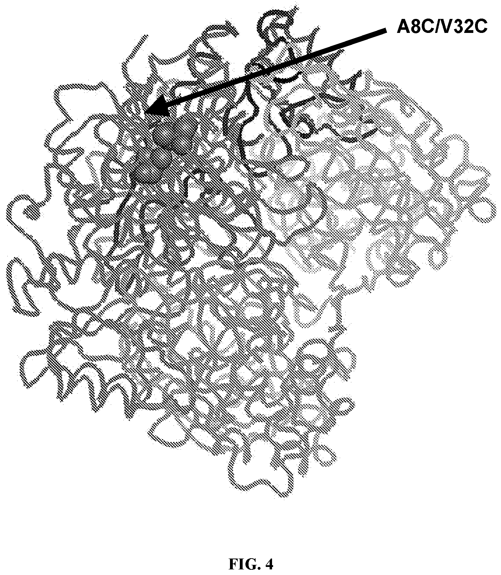

Three-dimensional protein structure analysis of tetrameric *E. coli* L-Asparaginase II was used to identify locations for introducing paired cysteine residues at or spatially near potential immunogenic or antigenic sites at which pegylation may shield the site without affecting its active site(s). Paired cysteines may be introduced within the same domain or across domains of the tetrameric protein. For example, Table 5 provides a listing of combinations of paired cysteine residues (C1 and C2) that may be introduced into tetrameric *E. coli* asparaginase within the same domain or across domains. By way of example, FIG. 4 shows the hypothetical three-dimensional structure of a variant *E. coli* asparaginase polypeptide having a cysteine pair introduced at position 8 and 32 of SEQ ID NO: 11 (SEQ ID NO: 12). The additional cysteine pair is identified in the figure.

**Table 5.**

| C1-C2 Paired cysteine substitution(s) for pegylation of *E*. *coli* asparaginase (based on mature amino acid sequence SEQ ID NO: 11) | | | |
|---|---|---|---|
| Cysteine substitution C1 | | Cysteine substitution C2 | |
| Subunit | Substitution | Subunit | Substitution |
| A | P2C | A | D81C |
| A | A8C | A | V32C |
| A | I13C | A | G125C |
| A | G15C | A | P117C |
| A | A20C | A | M121C |
| A | S23C | B | N184C |
| A | Y25C | A | P117C |
| A | V27C | A | G57C |
| A | G28C | A | N55C |
| A | Q59C | C | N248C |
| A | M61C | C | A221C |
| A | N62C | C | N222C |
| A | D63C | A | L211C |
| A | A70C | A | T102C |
| A | T89C | A | G113C |
| A | D90C | C | V244C |
| A | A96C | A | G157C |
| A | D100C | A | V146C |
| A | D100C | A | G157C |
| A | P108C | A | S141C |
| A | V112C | A | A132C |
| A | A114C | A | G125C |
| A | R116C | A | S120C |
| A | F127C | B | M121C |
| A | F127C | B | S122C |
| A | A132C | A | V149C |
| A | G145C | A | H197C |
| A | V146C | A | G157C |
| A | V154C | A | G180C |
| A | L155C | A | V160C |
| A | D159C | A | S173C |
| A | N164C | A | D167C |
| A | T165C | C | N246C |
| A | T165C | C | G276C |
| A | A169C | B | A169C |
| A | G180C | A | Y189C |
| A | N184C | B | S23C |
| A | K196C | A | D200C |
| A | P212C | A | D237C |
| A | G215C | A | L231C |
| A | A221C | C | M61C |
| A | N222C | C | N62C |
| A | A223C | A | Y250C |
| A | D225C | A | S252C |
| A | L226C | C | A230C |
| A | P227C | C | P227C |
| A | A230C | C | A230C |
| A | L231C | A | Y236C |
| A | G238C | A | A266C |
| A | A242C | A | P299C |
| A | V244C | C | D90C |
| A | G245C | C | D90C |
| A | G247C | A | R269C |
| A | N248C | C | Q59C |
| A | L249C | A | F254C |
| A | A260C | A | T265C |
| A | S270C | A | S294C |
| A | P274C | C | P274C |
| A | D286C | A | F291C |
| A | S294C | A | A302C |
| A | T313C | A | Q318C |
| A | D315C | A | Q318C |
| B | P2C | B | D81C |
| B | I4C | B | G82C |
| B | A8C | B | V32C |
| B | I13C | B | A114C |
| B | A14C | B | V30C |
| B | G15C | B | P117C |
| B | G17C | B | P117C |
| B | Y25C | B | P117C |
| B | V27C | B | G57C |
| B | G28C | B | N55C |
| B | V32C | B | G50C |
| B | S58C | B | T91C |
| B | Q59C | D | N248C |
| B | N62C | D | N222C |
| B | A70C | B | T102C |
| B | T89C | B | G113C |
| B | D90C | D | V244C |
| B | D90C | D | G245C |
| B | D100C | B | V146C |
| B | D100C | B | G157C |
| B | P108C | B | S141C |
| B | V112C | B | A132C |
| B | A114C | B | G125C |
| B | R116C | B | S120C |
| B | A132C | B | V149C |
| B | G145C | B | H197C |
| B | V146C | B | G157C |
| B | L155C | B | V160C |
| B | D159C | B | S173C |
| B | N164C | B | D167C |
| B | N164C | B | T170C |
| B | T165C | D | G276C |
| B | G180C | B | Y189C |
| B | K196C | B | D200C |
| B | P212C | B | D237C |
| B | P212C | B | L310C |
| B | N219C | B | V244C |
| B | A221C | D | M61C |
| B | N222C | D | N62C |
| B | A223C | B | Y250C |
| B | P227C | D | P227C |
| B | A230C | D | L226C |
| B | A230C | D | A230C |
| B | G238C | B | A266C |
| B | A242C | B | P299C |
| B | V244C | D | D90C |
| B | G245C | D | D90C |
| B | G247C | B | R269C |
| B | N248C | D | Q59C |
| B | L249C | B | F254C |
| B | A260C | B | T265C |
| B | S270C | B | S294C |
| B | S270C | B | A302C |
| B | P274C | D | P274C |
| B | G276C | D | T165C |
| B | S294C | B | A302C |
| B | L297C | B | K301C |
| B | D315C | B | Q318C |
| C | P2C | C | D81C |
| C | I4C | C | A46C |
| C | I6C | C | G50C |
| C | I13C | C | A114C |
| C | I13C | C | G125C |
| C | G15C | C | V27C |
| C | G16C | C | K29C |
| C | D18C | C | K22C |
| C | S19C | C | K22C |
| C | A20C | C | M121C |
| C | T89C | C | G113C |
| C | P108C | C | S141C |
| C | V112C | C | A132C |
| C | R116C | C | S120C |
| C | F127C | D | M121C |
| C | G145C | C | H197C |
| C | V146C | C | G157C |
| C | M150C | C | F171C |
| C | L155C | C | V160C |
| C | D156C | C | P193C |
| C | D159C | C | S173C |
| C | N164C | C | D167C |
| C | A169C | D | A169C |
| C | G180C | C | Y189C |
| C | N184C | D | S23C |
| C | K196C | C | D200C |
| C | P212C | C | D237C |
| C | G215C | C | L231C |
| C | A223C | C | Y250C |
| C | D225C | C | S252C |
| C | A228C | C | T256C |
| C | L231C | C | Y236C |
| C | G238C | C | A266C |
| C | A242C | C | P299C |
| C | G247C | C | R269C |
| C | A260C | C | T265C |
| C | A261C | C | G290C |
| C | S270C | C | S294C |
| C | D285C | C | K288C |
| C | D286C | C | F291C |
| C | S294C | C | A302C |
| C | L297C | C | A302C |
| C | T313C | C | Q318C |
| C | D315C | C | Q318C |
| D | P2C | D | D81C |
| D | I4C | D | G82C |
| D | A8C | D | V32C |
| D | I13C | D | A114C |
| D | G15C | D | P117C |
| D | G16C | D | K29C |
| D | G17C | D | Y25C |
| D | G17C | D | P117C |
| D | G17C | D | T119C |
| D | S19C | D | K22C |
| D | G28C | D | N55C |
| D | V32C | D | G50C |
| D | S58C | D | T91C |
| D | G82C | D | A136C |
| D | T89C | D | G113C |
| D | D100C | D | V146C |
| D | D100C | D | G157C |
| D | P108C | D | S141C |
| D | V112C | D | A132C |
| D | G145C | D | H197C |
| D | V146C | D | G157C |
| D | M150C | D | F171C |
| D | L155C | D | V160C |
| D | D159C | D | S173C |
| D | N164C | D | D167C |
| D | N164C | D | T170C |
| D | K196C | D | D200C |
| D | P212C | D | L310C |
| D | G215C | D | L231C |
| D | A223C | D | Y250C |
| D | L231C | D | Y236C |
| D | G238C | D | A266C |
| D | A242C | D | P299C |
| D | G247C | D | T278C |
| D | L249C | D | F254C |
| D | A260C | D | T265C |
| D | S270C | D | S294C |
| D | S271C | D | V273C |
| D | D285C | D | K288C |
| D | D286C | D | F291C |
| D | S294C | D | A302C |
| D | D315C | D | Q318C |

A third approach for introducing specific pegylation sites into *E. coli* asparaginase involves introducing one or more additional lysine residue(s) at surface-exposed locations at or spatially near potential immunogenic or antigenic sites, such as at or spatially near amino acid positions 55-58, 114-119, 201-207, and 252-258 of the mature amino acid sequence (SEQ ID NO: 11). For example, variant *E. coli* asparaginase polypeptides having one or more of the following lysine substitutions may be prepared: S58K, R116K, and/or D240K.

### Example 8

Variant *E. coli* K-12 asparaginase polypeptides ELSPAR^{®}, is an *E. coli* L-asparaginase indicated for the treatment of ALL and has been prescribed by veterinarians for treatment of lymphoma in dogs and cats. ELSPAR^{®} is derived from E. coli strain K-12 and has five cysteine residues. The precursor amino acid sequence is provided as SEQ ID NO: 13 and the mature sequence is provided by SEQ ID NO: 14. Three-dimensional protein structure analysis of tetrameric ELSPAR^{®} was used to identify that the two cysteines at positions 67 and 200 of SEQ ID NO: 14 likely form a disulfide linkage and the remaining three cysteines at positions 45, 67, and 242 of SEQ ID NO: 14 are likely unpaired. To reduce the likelihood of protein aggregation and/or reactivity to other molecules, one, two, or all three of the unpaired cysteines may be substituted with other amino acids, such as serine. For example, a variant *E. coli* asparaginase having a serine in place of the three unpaired cysteines at amino acid positions 45, 67, and 242 of SEQ ID NO: 14 may be prepared (SEQ ID NO: 15). Asparaginase polypeptides having one or more unpaired cysteine residues (e.g., polypeptides having the amino acid sequence of SEQ ID NO: 14, or having only one or two cysteine residues of SEQ ID NO: 14 substituted) may be used for conjugation.

## Claims

1. A variant asparaginase polypeptide comprising at least one amino acid substitution at an unpaired cysteine residue of a corresponding wildtype asparaginase polypeptide, wherein the at least one amino acid substitution comprises any amino acid other than cysteine.

2. The variant asparaginase polypeptide of claim 1, comprising at least one cysteine substitution at an amino acid position or at least one pair of cysteine substitutions at amino acid positions at or spatially near an immunogenic or antigenic site of the corresponding wildtype asparaginase polypeptide.

3. The variant asparaginase polypeptide of claim 2, wherein the at least one cysteine is partially embedded.

4. The variant asparaginase polypeptide of any one of the preceding claims, wherein the variant asparaginase polypeptide lacks a leader sequence.

5. The variant asparaginase polypeptide of any one of the preceding claims , wherein the immunogenic or antigenic site in the wildtype asparaginase polypeptide elicits an immune response in a human or in a companion animal species, optionally wherein the companion animal species is a canine, feline, or equine.

6. The variant asparaginase polypeptide of any one of the preceding claims , wherein between 5% and 25%, between 5% and 20%, between 5% and 15%, from 5% and 10%, between 10% and 20%, between 20% and 30%, or between 10% and 30% of the at least one cysteine is surface-exposed, as determined by a standard protein modeling software.

7. The variant asparaginase polypeptide of any one of the preceding claims , wherein the at least one cysteine is or the at least one pair of cysteines are 35 Angstroms (Å) or less from the immunogenic or antigenic site, as determined by three-dimensional protein structure analysis, optionally 30 Å or less, 25 Å or less, 20 Å or less, 15 Å or less, 10 Å or less, or 5 Å or less.

8. The variant asparaginase polypeptide of any one of the preceding claims, comprising an amino acid sequence that is at least 80%, at least 85%, at least 90%, at least 91%, at least 92%, at least 93%, at least 94%, at least 95%, at least 96%, at least 97%, at least 98%, or at least 99% identical to a wildtype asparaginase amino acid sequence, such as SEQ ID NO: 2, SEQ ID NO: 11, or SEQ ID NO: 14, optionally
wherein the wildtype asparaginase polypeptide is a wildtype *E. coli* asparaginase polypeptide, optionally wherein the wildtype asparaginase polypeptide comprises SEQ ID NO: 2, SEQ ID NO: 11, or SEQ ID NO: 14.

9. The variant asparaginase polypeptide of any one of claims 2 to 8, wherein (a) the immunogenic or antigenic site corresponds to amino acid position 55, 56, 57, 58, 114, 115, 116, 117, 118, 119, 201, 202, 203, 204, 205, 206, 207, 252, 253, 254, 255, 256, 257, or 258 of SEQ ID NO: 11; and/or (b) the at least one cysteine is located at a position corresponding to one or more amino acid position(s) selected from 51, 52, 118, 119, 196, 206, 285, and 311 of SEQ ID NO: 11; and/or (c) each of the at least one pair of cysteines is selected from C1-C2 paired cysteine substitution(s) listed in Table 5, optionally at a position corresponding to amino acid positions 116 and 120, amino acid positions 196 and 200, or amino acid positions 225 and 252 of SEQ ID NO: 11; and/or (d) wherein the unpaired cysteine residue is located at a position corresponding to amino acid position 45, 67, and/or 242 of SEQ ID NO: 14.

10. The variant asparaginase polypeptide of any one of the preceding claims, comprising the amino acid sequence of SEQ ID NO: 12 or SEQ ID NO: 15.

11. The variant asparaginase polypeptide of any one of claims 1 to 10 in monomeric, dimeric, or tetrameric form.

12. The variant asparaginase polypeptide of any one of claims 1 to 11, wherein the variant asparaginase polypeptide is modified, such as sialyated or pegylated, optionally thiol-pegylated or amine-pegylated.

13. An isolated nucleic acid encoding the variant asparaginase polypeptide of any one of claims 1 to 11.

14. A vector comprising the nucleic acid of claim 13.

15. A host cell comprising the vector of claim 14, optionally
wherein the cell is a prokaryotic cell, such as an *E. chrysanthemi* cell, an *E. coli* cell, or a *pseudomonas* cell; optionally the cell is a eukaryotic cell, such as a yeast cell.

16. A method of producing a variant asparaginase polypeptide, comprising culturing the host cell of claim 15 and optionally isolating the variant.

17. A pharmaceutical composition comprising the variant asparaginase polypeptide of any one of claims 1 to 12, and a pharmaceutically acceptable carrier.

18. The variant asparaginase polypeptide of any one of claims 1 to 12 or the pharmaceutical composition of claim 17 for use in a method of treatment, wherein the method comprises administering the polypeptide or composition to a subject.

19. The variant asparaginase polypeptide for use or the pharmaceutical composition for use according to claim 18, for use in a method of treating lymphoma or an acute lymphoblastic leukemia.

20. The variant asparaginase polypeptide for use or the pharmaceutical composition for use according to claims 18 or 19, wherein the subject is a human or a companion animal species.

21. The variant asparaginase polypeptide for use or the pharmaceutical composition for use according to claim 20, wherein the companion animal species is canine, feline, or equine.

## Patentansprüche

1. Asparaginase-Polypeptidvariante, die zumindest eine Aminosäuresubstitution an einem ungepaarten Cysteinrest eines entsprechenden Asparaginase-Wildtyppolypeptids umfasst, wobei die zumindest eine Aminosäuresubstitution eine beliebige Aminosäure umfasst, die nicht Cystein ist.

2. Asparaginase-Polypeptidvariante nach Anspruch 1, die zumindest eine Cysteinsubstitution an einer Aminosäureposition oder zumindest ein Paar von Cysteinsubstitutionen an Aminosäurepositionen an oder räumlich nahe an einer immunogenen oder antigenen Stelle des entsprechenden Asparaginase-Wildtyppolypeptids umfasst.

3. Asparaginase-Polypeptidvariante nach Anspruch 2, wobei das zumindest eine Cystein teilweise eingebettet ist.

4. Asparaginase-Polypeptidvariante nach einem der vorangegangenen Ansprüche, wobei der Asparaginase-Polypeptidvariante eine Leader-Sequenz fehlt.

5. Asparaginase-Polypeptidvariante nach einem der vorangegangenen Ansprüche, wobei die immunogene oder antigene Stelle in dem Asparaginase-Wildtyppolypeptid eine Immunantwort in einem Menschen oder in einer Haustierspezies auslöst, wobei die Haustierspezies gegebenenfalls ein Hund, eine Katze oder ein Pferd ist.

6. Asparaginase-Polypeptidvariante nach einem der vorangegangenen Ansprüche, wobei zwischen 5 % und 25 %, zwischen 5 % und 20 %, zwischen 5 % und 15 %, von 5 % und 10 %, zwischen 10 % und 20 %, zwischen 20 % und 30 % oder zwischen 10 % und 30 % des zumindest einen Cysteins oberflächenexponiert sind, wie durch eine Standard-Proteinmodellierungssoftware bestimmt.

7. Asparaginase-Polypeptidvariante nach einem der vorangegangenen Ansprüche, wobei sich das zumindest eine Cystein oder das zumindest eine Paar von Cysteinen 35 Ångström (Å) oder weniger, wie durch dreidimensionale Proteinstrukturanalyse bestimmt, gegebenenfalls 30 Å oder weniger, 25 Å oder weniger, 20 Å oder weniger, 15 Å oder weniger, 10 Å oder weniger oder 5 Å oder weniger von der immunogenen oder antigenen Stelle entfernt befindet.

8. Asparaginase-Polypeptidvariante nach einem der vorangegangenen Ansprüche, umfassend eine Aminosäuresequenz, die eine Identität von zumindest 80 %, zumindest 85 %, zumindest 90 %, zumindest 91 %, zumindest 92 %, zumindest 93 %, zumindest 94 %, zumindest 95 %, zumindest 96 %, zumindest 97 %, zumindest 98 % oder zumindest 99 % mit einer Asparaginase-Wildtypaminosäuresequenz, wie z. B. SEQ ID NO: 2, SEQ ID NO: 11 oder SEQ ID NO: 14, aufweist,
wobei das Asparaginase-Wildtyppolypeptid gegebenenfalls ein E.-coli-Asparaginase-Wildtyppolypeptid ist, wobei das Asparaginase-Wildtyppolypeptid gegebenenfalls SEQ ID NO: 2, SEQ ID NO: 11 oder SEQ ID NO: 14 umfasst.

9. Asparaginase-Polypeptidvariante nach einem der Ansprüche 2 bis 8, wobei (a) die immunogene oder antigene Stelle Aminosäureposition 55, 56, 57, 58, 114, 115, 116, 117, 118, 119, 201, 202, 203, 204, 205, 206, 207, 252, 253, 254, 255, 256, 257 oder 258 von SEQ ID NO: 11 entspricht; und/oder (b) das zumindest eine Cystein an einer Position vorhanden ist, die einer oder mehreren Aminosäurepositionen entspricht, die aus 51, 52, 118, 119, 196, 206, 285 und 311 von SEQ ID NO: 11 ausgewählt sind; und/oder (c) jedes aus dem zumindest einen Paar von Cysteinen aus C1-C2-gepaarten Cystein-Substitutionen, die in Tabelle 5 aufgeführt sind, gegebenenfalls an einer Position entsprechend Aminosäurepositionen 116 und 120, Aminosäurepositionen 196 und 200 oder Aminosäurepositionen 225 und 252 von SEQ ID NO: 11, ausgewählt ist; und/oder (d) wobei der ungepaarte Cysteinrest an einer Position entsprechend Aminosäureposition 45, 67 und/oder 242 von SEQ ID NO: 14 vorhanden ist.

10. Asparaginase-Polypeptidvariante nach einem der vorangegangenen Ansprüche, umfassend die Aminosäuresequenz von SEQ ID NO: 12 oder SEQ ID NO: 15.

11. Asparaginase-Polypeptidvariante nach einem der Ansprüche 1 bis 10 in monomerer, dimerer oder tetramerer Form.

12. Asparaginase-Polypeptidvariante nach einem der Ansprüche 1 bis 11, wobei die Asparaginase-Polypeptidvariante modifiziert, z. B. sialyiert oder pegyliert, gegebenenfalls Thiolpegyliert oder Amin-pegyliert, ist.

13. Isolierte Nucleinsäure, die für eine Asparaginase-Polypeptidvariante nach einem der Ansprüche 1 bis 11 kodiert.

14. Vektor, umfassend eine Nucleinsäure nach Anspruch 13.

15. Wirtszelle, umfassend einen Vektor nach Anspruch 14, wobei die Zelle gegebenenfalls eine prokaryotische Zelle, wie z. B. eine E.-chrysanthemi-Zelle, eine E.-coli-Zelle oder eine Pseudomonas-Zelle, ist; wobei die Zelle gegebenenfalls eine eukaryotische Zelle, wie z. B. eine Hefezelle, ist.

16. Verfahren zur Herstellung einer Asparaginase-Polypeptidvariante, umfassend das Kultivieren einer Wirtszelle nach Anspruch 15 und gegebenenfalls das Isolieren der Variante.

17. Pharmazeutische Zusammensetzung, umfassend eine Asparaginase-Polypeptidvariante nach einem der Ansprüche 1 bis 12 und einen pharmazeutisch annehmbaren Träger.

18. Asparaginase-Polypeptidvariante nach einem der Ansprüche 1 bis 12 oder pharmazeutische Zusammensetzung nach Anspruch 17 zur Verwendung in einem Verfahren zur Behandlung, wobei das Verfahren das Verabreichen des Polypeptids oder der Zusammensetzung an ein Individuum umfasst.

19. Asparaginase-Polypeptidvariante zur Verwendung oder pharmazeutische Zusammensetzung zur Verwendung nach Anspruch 18 zur Verwendung in einem Verfahren zur Behandlung eines Lymphoms oder von akuter lymphoblastischer Leukämie.

20. Asparaginase-Polypeptidvariante zur Verwendung oder pharmazeutische Zusammensetzung zur Verwendung nach Anspruch 18 oder 19, wobei das Individuum ein Mensch oder eine Haustierspezies ist.

21. Asparaginase-Polypeptidvariante zur Verwendung oder pharmazeutische Zusammensetzung zur Verwendung nach Anspruch 20, wobei die Haustierspezies ein Hund, eine Katze oder ein Pferd ist.

## Revendications

1. Polypeptide d'asparaginase variant comprenant au moins une substitution d'acide aminé au niveau d'un résidu de cystéine non apparié d'un polypeptide d'asparaginase de type sauvage correspondant, dans lequel la au moins une substitution d'acide aminé comprend un acide aminé quelconque autre que la cystéine.

2. Polypeptide d'asparaginase variant selon la revendication 1, comprenant au moins une substitution de cystéine en une position d'acide aminé ou au moins une paire de substitutions de cystéine en des positions d'acide aminé au niveau ou à proximité spatiale d'un site immunogène ou antigénique du polypeptide d'asparaginase de type sauvage correspondant.

3. Polypeptide d'asparaginase variant selon la revendication 2, dans lequel la au moins une cystéine est partiellement incorporée.

4. Polypeptide d'asparaginase variant selon l'une quelconque des revendications précédentes, dans lequel le polypeptide d'asparaginase variant est dépourvu d'une séquence leader.

5. Polypeptide d'asparaginase variant selon l'une quelconque des revendications précédentes, dans lequel le site immunogène ou antigénique dans le polypeptide d'asparaginase de type sauvage provoque une réponse immunitaire chez un humain ou chez une espèce animale de compagnie, facultativement dans lequel l'espèce animale de compagnie est un chien, un félin ou un équidé.

6. Polypeptide d'asparaginase variant selon l'une quelconque des revendications précédentes, dans lequel entre 5 % et 25 %, entre 5 % et 20 %, entre 5 % et 15 %, entre 5 % et 10 %, entre 10 % et 20 %, entre 20 % et 30 %, ou entre 10 % et 30 % de la au moins une cystéine sont exposées en surface, comme déterminé par un logiciel de modélisation de protéines standard.

7. Polypeptide d'asparaginase variant selon l'une quelconque des revendications précédentes, dans lequel la au moins une cystéine est ou la au moins une paire de cystéines sont à 35 Angströms (Å) ou moins du site immunogène ou antigénique, comme déterminé par analyse de structure protéique en tridimensionnelle, facultativement à 30 Å ou moins, à 25 Å ou moins, à 20 Å ou moins, à 15 Å ou moins, à 10 Å ou moins, ou à 5 Å ou moins.

8. Polypeptide d'asparaginase variant selon l'une quelconque des revendications précédentes, comprenant une séquence d'acides aminés qui est au moins 80%, au moins 85%, au moins 90 %, au moins 91 %, au moins 92 %, au moins 93 %, au moins 94 %, au moins 95 %, au moins 96 %, au moins 97 %, au moins 98%, ou au moins 99% identique à une séquence d'acides aminés d'asparaginase de type sauvage, telle que SEQ ID NO : 2, SEQ ID NO : 11 ou SEQ ID NO : 14, facultativement
dans lequel le polypeptide d'asparaginase de type sauvage est un polypeptide d'asparaginase d'E. *coli* de type sauvage, facultativement dans lequel le polypeptide d'asparaginase de type sauvage comprend SEQ ID NO : 2, SEQ ID NO : 11 ou SEQ ID NO : 14.

9. Polypeptide d'asparaginase variant selon l'une quelconque des revendications 2 à 8, dans lequel (a) le site immunogène ou antigénique correspond à la position d'acide aminé 55, 56, 57, 58, 114, 115, 116, 117, 118, 119, 201, 202, 203, 204, 205, 206, 207, 252, 253, 254, 255, 256, 257 ou 258 de SEQ ID NO : 11 ; et/ou (b) la au moins une cystéine est située en une position correspondant à une ou plusieurs positions d'acide aminé choisies parmi 51, 52, 118, 119, 196, 206, 285 et 311 de SEQ ID NO : 11 ; et/ou (c) chacune de la au moins une paire de cystéines est choisie parmi la ou les substitutions de cystéine appariées en C1-C2 énumérées dans le tableau 5, facultativement en une position correspondant aux positions d'acides aminés 116 et 120, aux positions d'acides aminés 196 et 200, ou aux positions d'acides aminés 225 et 252 de SEQ ID NO : 11 ; et/ou (d) dans lequel le résidu de cystéine non apparié est situé en une position correspondant à la position d'acide aminé 45, 67 et/ou 242 de SEQ ID NO : 14.

10. Polypeptide d'asparaginase variant selon l'une quelconque des revendications précédentes, comprenant la séquence d'acides aminés de SEQ ID NO : 12 ou SEQ ID NO : 15.

11. Polypeptide d'asparaginase variant selon l'une quelconque des revendications 1 à 10 sous forme monomère, dimère ou tétramère.

12. Polypeptide d'asparaginase variant selon l'une quelconque des revendications 1 à 11, dans lequel le polypeptide d'asparaginase variant est modifié, tel que sialyé ou pégylé, facultativement pégylé par thiol ou pégylé par amine.

13. Acide nucléique codant pour un anticorps humanisé selon l'une quelconque des revendications 1 à 11.

14. Vecteur comprenant l'acide nucléique selon la revendication 13.

15. Cellule hôte comprenant le vecteur de la revendication 14, facultativement
dans laquelle la cellule est une cellule procaryote, telle qu'une cellule d'*E*. *chrysanthemi,* une cellule d'*E*. *coli* ou une cellule de *pseudomonas* ; facultativement, la cellule est une cellule eucaryote, telle qu'une cellule de levure.

16. Procédé de production d'un polypeptide d'asparaginase variant, comprenant la mise en culture de la cellule hôte de la revendication 15 et facultativement l'isolement du variant.

17. Composition pharmaceutique comprenant l'anticorps selon l'une quelconque des revendications 1 à 12 et un excipient pharmaceutiquement acceptable.

18. Polypeptide d'asparaginase variant selon l'une quelconque des revendications 1 à 12 ou composition pharmaceutique selon la revendication 17 pour utilisation dans un procédé de traitement, dans lequel le procédé comprend l'administration du polypeptide ou de la composition à un sujet.

19. Polypeptide d'asparaginase variant pour utilisation ou composition pharmaceutique pour utilisation selon la revendication 18, pour utilisation dans un procédé de traitement d'un lymphome ou d'une leucémie lymphoblastique aiguë.

20. Polypeptide d'asparaginase variant pour utilisation ou composition pharmaceutique pour utilisation selon les revendications 18 ou 19, dans lequel le sujet est une espèce humaine ou animale de compagnie.

21. Polypeptide d'asparaginase variant pour utilisation ou composition pharmaceutique pour utilisation selon la revendication 20, dans lequel l'espèce d'animal de compagnie est un chien, un félin ou un équidé.
